(19) 

Europäisches
Patentamt
European
Patent Office
Office européen
des brevets

(11) **EP 2 890 345 B1**

(12) **EUROPEAN PATENT SPECIFICATION**

(45) Date of publication and mention
of the grant of the patent:
**06.12.2017   Bulletin 2017/49**

(21) Application number: **13770974.7**

(22) Date of filing: **30.08.2013**

(51) Int Cl.:
*A61F 13/53* *(2006.01)*      *A61F 13/537* *(2006.01)*

(86) International application number:
**PCT/JP2013/005133**

(87) International publication number:
**WO 2014/034132 (06.03.2014 Gazette 2014/10)**

(54) **ABSORBENT BODY AND ABSORBENT ARTICLE USING THE SAME**

ABSORBIERENDEN KÖRPER UND VERWENDUNG IN ABSORBIERENDEN ARTIKEL

CORPS ABSORBANT ET ARTICLE ABSORBANT UTILISANT CE CORPS ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **31.08.2012   JP 2012192185**

(43) Date of publication of application:
**08.07.2015   Bulletin 2015/28**

(73) Proprietor: **Livedo Corporation
ShikokuChuo-shi
Ehime 799-0122 (JP)**

(72) Inventors:
  • **OTA, Yoshihisa
Mima-gun
Tokushima 779-4104 (JP)**

  • **NISHIDA, Motoko
Mima-gun
Tokushima 779-4104 (JP)**

(74) Representative: **Gulde & Partner
Patent- und Rechtsanwaltskanzlei mbB
Wallstraße 58/59
10179 Berlin (DE)**

(56) References cited:
**EP-A1- 1 518 567      EP-A1- 1 579 831
EP-A2- 0 202 125      US-A- 5 419 956
US-A1- 2004 019 342**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

[Technical Field]

The present invention relates to an absorbent body used in absorbent articles such as incontinence pads, disposable diapers, and sanitary napkins; and the present invention particularly relates to an improvement technology regarding absorption performance of an absorbent body.

[Background Art]

Absorbent articles such as incontinence pads, disposable diapers, and sanitary napkins include an absorbent body for absorbing and retaining body fluid excreted from body such as urine and menstrual blood. The absorbent body generally includes a water absorbent resin powder, and body fluid or the like is absorbed and retained in the water absorbent resin powder inside the absorbent body. There have been proposals of water absorbent articles having an absorbent body with improved absorption rate of body fluid or the like.

For example, JP 2011-055959A proposes an absorbent article comprising a liquid permeable top sheet, a liquid impermeable back sheet, and an absorbent core interposed therebetween, wherein a second sheet formed from a nonwoven fabric having a hollow fiber is interposed between the top sheet and the absorbent core.

JP 2002-528302 A proposes an absorption product comprising: (a) a top sheet comprising an aperture polymeric film web having a first surface, and a second surface generally parallel to and spaced from the first surface, and a plurality of fluid passageways extending between the first surface and the second surface in fluid communication with one another, the web being formed of a polymeric film comprising at least one bulk modified layer, the bulk modified layer comprising a substantially homogeneous stabilized dispersion of a hydrophobic additive in a polymer material; (b) a back sheet peripherally joined with the top sheet; and (c) an absorbent core positioned between the second surface of the top sheet and the back sheet.

JP 2006-524112 A proposes an absorbent article comprising: a top sheet; a back sheet; and an intermediate layer between the top sheet and the back sheet; wherein at least one of the top sheet, back sheet, and intermediate layer comprises a three-dimensional vacuum formed film with a male side void volume of at least 350 cc/m$^2$; and the absorbent article having a first minute decrease in temperature of at least 8 degrees F in a Third Insult Test.

EP 1 518 567 A1 discloses an absorbent core comprising a fluid acquisition zone which serves to quickly collect and temporarily hold discharged body fluid. For this purpose, the fluid acquisition zone comprises super absorbent polymer particles.

EP 1 579 831 A1 discloses a fluid acquisition/distribution layer comprising several different materials including nonwoven or woven webs of synthetic fibers including polyester, polypropylene, or polyethylene.

US 2004/0019342 A1 discloses a liquid-diffusing member displaying a capillary absorption capacity of not less than 10 g/g at a height of 0 cm and a liquid-acquiring member displaying a capillary absorption capacity of not less than 5 g/g at the same height. That is, the liquid-diffusing and liquid-acquiring members are both materials sucking and absorbing a liquid by capillary action.

US 5,419,956 A discloses an absorbent core comprising a storage zone and an acquisition zone provided therein. Both, the acquisition zone and the storage zone comprise particles of a superabsorbent hydrogel-forming material.

[Summary of Invention]

[Technical Problem]

The under-load liquid passing properties have been found to be inferior in all of the nonwoven fabric, the polymeric film web and the three-dimensional vacuum formed film with a void volume (cf. JP 2011-055959 A, JP 2002-528302 A, JP 2006-524112 A), which have been used conventionally for improving a water absorbent article's absorption rate of body fluid or the like. Thus, the absorption rate of body fluid or the like has been insufficient when a load is applied on the water absorbent article. Furthermore, in particular, films having holes such as those used in JP 2002-528302 A and JP 2006-524112 A have a problem that liquid may easily remain in the voids within the film and cause rashes.

The present invention has been made in view of the above described circumstances; and an objective of the present invention is to provide an absorbent body having an excellent absorption rate of body fluid or the like and excellent dryness after absorbing the body fluid or the like, and an absorbent article using the absorbent body.

[Solution to Problem]

The present invention is directed to an absorbent body including a water absorbent resin powder, the absorbent body comprising a layer having formed at least partially thereon a diffusion region where a diffusibility improvement material having an under-load liquid passing rate of 15 seconds or less (determined by a test method as described below) is disposed, wherein the diffusibility improvement material comprises resin particles having a particle diameter of from 0.05 mm to 10 mm. With the layer having the diffusion region formed thereon, body fluid or the like absorbed by the absorbent body is diffused in the thickness direction and the planar direction of the layer in the diffusion region, and then absorbed in the water absorbent resin powder. Therefore, since the body fluid or the like diffused in the diffusion region is absorbed by the water absorbent resin powder in various directions, gel-blocking of the water absorbent resin powder is suppressed, and all of the water absorbent resin powder included in the absorbent body can contribute to the absorption of the body fluid or the like. In addition, since the diffusion region has disposed thereon the diffusibility improvement material whose under-load liquid passing rate is 15 seconds or less, excellent liquid passing property can be exerted even under load. As a result, the absorbent body of the present invention has an excellent absorption rate of body fluid or the like.

The absorbent body preferably has a diffusion-and-water absorption layer including the diffusion region and a water absorption region where the water absorbent resin powder is disposed. The body fluid or the like taken into the diffusion region is immediately absorbed by the water absorption region formed within the same layer. As a result, the absorption rate of the body fluid or the like is further improved. The absorbent body is preferably formed from a single layer of the diffusion-and-water absorption layer.

An area ratio of the diffusion region with respect to the surface of the diffusion-and-water absorption layer is preferably 5% or more and is preferably 70% or less. A suitable planar view shape of the diffusion region is a circular shape, an elliptical shape, a polygonal shape, a polygonal shape having rounded corners, or a slit shape. Furthermore, the diffusion region having the circular shape, the elliptical shape, the polygonal shape, or the polygonal shape having rounded corners is preferably formed starting from an approximately center part of the diffusion-and-water absorption layer in a longitudinal direction, toward one side thereof in the longitudinal direction.

The absorbent body preferably has at least two or more layers of a diffusion layer only having a diffusion region and a water absorption layer only having a water absorption region below the diffusion layer. By having such a configuration, the body fluid or the like taken into the absorbent body is diffused in a planar direction within the diffusion layer, and is then absorbed by the water absorption layer disposed below the diffusion layer. As a result, since the body fluid or the like is taken in from a broad range of the top surface of the water absorption layer, the absorption rate is further improved. In this embodiment, the water absorption layer preferably includes a water absorbent resin powder satisfying the following requirements:

Absorption ratio: 50 g/g to 70 g/g
Water retention amount: 25 g/g to 60 g/g
Absorption rate determined by a vortex method: 5 seconds to 60 seconds.

The absorbent body preferably has at least two or more layers of a diffusion-and-water absorption layer having formed thereon a slit shaped diffusion region, and a water absorption layer only having a water absorption region below the diffusion-and-water absorption layer. By having such a configuration, the body fluid or the like taken into the diffusion region is immediately absorbed by the water absorption region formed within the same layer, and the body fluid or the like diffused in the planar direction within the diffusion region is also absorbed by the water absorption layer disposed below the diffusion region. As a result, the absorption rate becomes fast, and absorptive capacity is further improved. In this embodiment, the water absorption layer preferably includes a water absorbent resin powder satisfying the following requirements:

Absorption ratio: 40 g/g to 70 g/g
Water retention amount: 30 g/g to 50 g/g.

The present invention also includes an absorbent article having the above absorbent body.

**Advantageous Effects of the Invention**

The absorbent body and the absorbent article of the present invention have an excellent absorption rate of body fluid or the like, and excellent dryness after absorbing the body fluid or the like.

**Brief Description of Drawings**

[fig.1]FIG. 1 is a schematic perspective view of an absorbent body according to a preferable Embodiment 1 of the present invention.

[fig.2]FIG. 2 is a cross sectional view along line X-X in FIG. 1.

[fig.3]FIG. 3 is a schematic perspective view of an absorbent body according to a preferable Embodiment 2 of the present invention.

[fig.4]FIG. 4 is a cross sectional view along line Y-Y in FIG. 3.

[fig.5]FIG. 5 is a schematic sectional view of an absorbent body according to a preferable Embodiment 3 of the present invention.

[fig.6]FIG. 6 is a cross sectional view along line Z-Z in FIG. 5.

[fig.7]FIG. 7 is a plan view showing how a diffusion region is formed in a diffusion-and-water absorption layer.

[fig.8]FIG. 8 is a schematic plan view of an absorbent article according to a preferable embodiment of the present invention.

[fig.9]FIG. 9 is a cross sectional view along line V-V in FIG. 8.

[fig.10]FIG. 10 is a schematic diagram for describing a method for measuring an under-load liquid passing rate of a diffusibility improvement material.

**Description of Embodiments**

The present invention provides an absorbent body including a water absorbent resin powder, the absorbent body comprising a layer having formed at least partially thereon a diffusion region where a diffusibility improvement material having an under-load liquid passing rate of 15 seconds or less is disposed. With the layer having the diffusion region formed thereon, body fluid or the like absorbed by the absorbent body is diffused in the thickness direction and the planar direction of the layer in the diffusion region, and then absorbed in the water absorbent resin powder. Thus, since the body fluid or the like diffused in the diffusion region is absorbed by the water absorbent resin powder in various directions, gel-blocking of the water absorbent resin powder is suppressed, and all of the water absorbent resin powder included in the absorbent body can contribute to the absorption of the body fluid or the like. In addition, since the diffusion region has disposed thereon the diffusibility improvement material whose under-load liquid passing rate is 15 seconds or less, excellent liquid passing property can be exerted even under load. Therefore, the absorbent body of the present invention has excellent absorption rate of body fluid or the like, and excellent dryness after absorbing the body fluid or the like.

Water absorbent resin powder

First, description will be provided for a water absorbent resin powder used in the present invention. The water absorbent resin powder may be contained in a layer including the diffusion region, or may be contained in a layer that does not include the diffusion region. The water absorbent resin powder used in the present invention preferably includes a crosslinked polymer (A) mainly composed of acrylic acid and having carboxyl groups thereof being at least partially neutralized. The percentage content of the acrylic acid component forming the crosslinked polymer is preferably 90 mass% or more, more preferably 95 mass% or more, and is preferably 99 mass% or less, more preferably 97 mass% or less. If the percentage content of the acrylic acid component is within the above described range, the obtained water absorbent resin powder can easily express a desired absorption performance.

Examples of cations for neutralizing at least a part of the carboxyl groups of the crosslinked polymer (A) include, but not particularly limited to, alkali metal ions such as lithium, sodium, and potassium; and alkaline earth metal ions such as magnesium and calcium. Of those described above, at least a part of the carboxyl groups of the crosslinked polymer is preferably neutralized with the sodium ion. It should be noted that, with regard to neutralization of the carboxyl groups of the crosslinked polymer, neutralization may be conducted on the carboxyl groups of the crosslinked polymer which has been obtained by polymerization or neutralization may be conducted in advance on a monomer which is then used for forming the crosslinked polymer.

The degree of neutralization of the carboxyl groups of the crosslinked polymer is preferably 60 mole% or more, and more preferably 65 mole% or more. This is because there are cases where the absorption performance of the obtained water-absorbent resin powder deteriorates if the degree of neutralization is too low. Furthermore, there is no particular limitation on the upper limit of the degree of neutralization, and all the carboxyl groups may be neutralized. It should be noted that the degree of neutralization is obtained by the following formula.

$$\text{Degree of neutralization (mole\%)} = 100 \times [\text{Number of moles of neutralized carboxyl groups in the crosslinked polymer}] / [\text{Total number of moles of the carboxyl groups in the crosslinked polymer (including neutralized and unneutralized groups)}]$$

The crosslinked polymer (A) preferably includes those obtained by polymerization of the unsaturated monomer composition containing a water-soluble ethylenically unsaturated monomer (a1) and/or a hydrolyzable monomer (a2) producing the water-soluble ethylenically unsaturated monomer (a1) by hydrolysis and an internal crosslinking agent (b).

The water-soluble ethylenically unsaturated monomer (a1) is not particularly limited, but a monomer having at least one water-soluble substituent and an ethylenically unsaturated group, or the like can be used. The water-soluble monomer means a monomer having a property of being dissolved in an amount of at least 100 g in 100 g of water at 25 degrees centigrade. In addition, the hydrolyzable monomer (a2) is hydrolyzed with water at 50 degrees centigrade, by the action of a catalyst (an acid, a base, or the like) where necessary, to produce the water-soluble ethylenically unsaturated monomer (a1). The hydrolysis of the hydrolyzable monomer (a2) may be conducted during or after the polymerization of the crosslinked polymer (A) or both during and after the polymerization of the crosslinked polymer (A). However, the hydrolysis of the hydrolyzable monomer (a2) is preferably conducted after the polymerization of the crosslinked polymer (A) in light of the molecular weight of the obtained water-absorbent resin powder and the like.

Examples of the water-soluble substituent include a carboxyl group, a sulfo group, a sulfoxy group, a phosphono group, a hydroxyl group, a carbamoyl group, an amino group, or salts thereof and an ammonium salt. A salt of a carboxyl group (a carboxylate), a salt of a sulfo group (a sulfonate), and an ammonium salt are preferred. In addition, examples of the salts include salts of alkali metal such as lithium, sodium, and potassium and salts of alkaline earth metal such as magnesium and calcium. The ammonium salt may be any of salts of primary to tertiary amines or a quaternary ammonium salt. Of these salts, in light of absorption properties, alkali metal salts and ammonium salts are preferred, and alkali metal salts are more preferred, and sodium salts are further preferred.

As the water-soluble ethylenically unsaturated monomer having a carboxyl group and/or a salt thereof, an unsaturated carboxylic acid having 3 to 30 carbon atoms and/ or a salt thereof are preferred. Specific examples of the water-soluble ethylenically unsaturated monomer having a carboxyl group and/or a salt thereof include unsaturated monocarboxylic acids and/or salts thereof such as (meth)acrylic acid, (meth)acrylic acid salt, crotonic acid, and cinnamic acid; unsaturated dicarboxylic acids and/or salts thereof such as maleic acid, maleate, fumaric acid, citraconic acid, and itaconic acid; and monoalkyl (1 to 8 carbon atoms) esters of unsaturated dicarboxylic acids and/or salts thereof such as maleic acid monobutyl ester, fumaric acid monobutyl ester, ethylcarbitol monoester of maleic acid, ethylcarbitol monoester of fumaric acid, citraconic acid monobutyl ester, and itaconic acid glycol monoester. It is noted that in the description of the present invention, "(meth)acrylic" means "acrylic" and/or "methacrylic".

As a water-soluble ethylenically unsaturated monomer having a sulfo group and/or a salt thereof, a sulfonic acid having 2 to 30 carbon atoms and/or a slat thereof are preferred. Specific examples of the water-soluble ethylenically unsaturated monomer having a sulfo group and/or a salt thereof include aliphatic or aromatic vinyl sulfonic acids such as vinyl sulfonic acid, (meth)allyl sulfonic acid, styrene sulfonic acid, and alpha-methyl styrene sulfonic acid; (meth)acryloyl-containing alkyl sulfonic acids such as (meth)acryloxy propyl sulfonic acid, 2-hydroxy-3-(meth)acryloxy propyl sulfonic acid, 2-(meth)acryloylamino-2,2-dimethylethane sulfonic acid, 3-(meth)acryloxyethane sulfonic acid, 2-(meth)acrylamide-2-methylpropane sulfonic acid, and 3-(meth)acrylamide-2-hydroxypropane sulfonic acid; and alkyl(meth)allyl sulfosuccinate.

Examples of a water-soluble ethylenically unsaturated monomer having a sulfoxy group and/or a salt thereof include sulfate ester of hydroxy alkyl (meth)acrylate; and sulfate ester of polyoxyalkylene mono(meth)acrylate.

Examples of a water-soluble ethylenically unsaturated monomer having a phosphono group and/or a salt thereof include phosphate monoesters of (meth)acrylic acid hydroxyalkyl, phosphate diesters of (meth)acrylic acid hydroxyalkyl, and (meth)acrylic acid alkylphosphonic acids.

Examples of a water-soluble ethylenically unsaturated monomer having a hydroxyl group include mono-ethylenically unsaturated alcohols having 3 to 15 carbon atoms such as (meth)allyl alcohol and (meth)propenyl alcohol; mono-ethylenically unsaturated carboxylates or mono-ethylenically unsaturated ethers of bivalent to hexavalent polyols such as alkylene glycol having 2 to 20 carbon atoms, glycerin, sorbitan, diglycerin, pentaerythritol, and polyalkylene (2 to 4 carbon atoms) glycol (weight average molecular weight: 100 to 2000). Specific examples of them include hydroxyethyl(meth)acrylate, hydroxypropyl(meth)acrylate, triethyleneglycol(meth)acrylate, and poly-oxyethylene-oxypropylene mono(meth)allyl ether.

Examples of a water-soluble ethylenically unsaturated monomer having a carbamoyl group include (meth)acrylamide; N-alkyl (1 to 8 carbon atoms) (meth)acrylamides such as N-methyl acrylamide; N,N-dialkyl (alkyl having 1 to 8 carbon atoms) acrylamides such as N,N-dimethyl acrylamide and N,N-di-n- or i-propyl acrylamide; N-hydroxyalkyl (1 to 8 carbon atoms) (meth)acrylamides such as N-methylol (meth)acrylamide and N-hydroxyethyl (meth)acrylamide; and N,N-dihydroxyalkyl (1 to 8 carbon atoms) (meth)acrylamides such as N,N-dihydroxyethyl (meth)acrylamide. As an unsaturated monomer having a group composed of an amide, in addition to them, vinyl lactams having 5 to 10 carbon atoms (N-vinyl pyrrolidone, etc.) and the like can also be used.

Examples of a water-soluble ethylenically unsaturated monomer having an amino group include an amino group-containing ester of a mono-ethylenically unsaturated mono- or di-carboxylic acid and an amino group-containing amide of a mono-ethylenically unsaturated mono- or di-carboxylic acid. As the amino group-containing ester of a mono-ethylenically unsaturated mono- or di-carboxylic acid, dialkylaminoalkyl(meth)acrylate, di(hydroxyalkyl)aminoalkyl ester, morpholinoalkyl ester, and the like can be used, and examples thereof include dimethylaminoethyl (meth)acrylate, diethylamino (meth)acrylate, morpholinoethyl (meth)acrylate, dimethylaminoethyl fumarate, and dimethylaminoethyl malate. As the amino group-containing amide of a mono-ethylenically unsaturated mono- or di-carboxylic acid, monoalkyl (meth)acrylamide is preferred, and examples thereof include dimethylaminoethyl (meth)acrylamide and diethylaminoethyl (meth)acrylamide. As the water-soluble ethylenically unsaturated monomer having an amino group, in addition to them, vinylpyridines such as 4-vinylpyridine and 2-vinylpyridine can also be used.

The hydrolyzable monomer (a2) producing the water-soluble ethylenically unsaturated monomer (a1) by hydrolysis is not particularly limited, but an ethylenically unsaturated monomer having at least one hydrolyzable substituent that becomes a water-soluble substituent by hydrolysis is preferred. Examples of the hydrolyzable substituent include a group containing an acid anhydride, a group containing an ester linkage, and a cyano group.

As an ethylenically unsaturated monomer having a group containing an acid anhydride, an unsaturated dicarboxylic anhydride having 4 to 20 carbon atoms is used, and examples thereof include maleic anhydride, itaconic anhydride, and citraconic anhydride. Examples of an ethylenically unsaturated monomer having a group containing an ester linkage include lower alkyl esters of mono-ethylenically unsaturated carboxylic acids such as methyl (meth)acrylate and ethyl (meth)acrylate; and esters of mono-ethylenically unsaturated alcohols such as vinyl acetate and (meth)allyl acetate. Examples of an ethylenically unsaturated monomer having a cyano group include vinyl group-containing nitrile compounds having 3 to 6 carbon atoms such as (meth)acrylonitrile and 5-hexenenitrile.

As the water-soluble ethylenically unsaturated monomer (a1) and the hydrolyzable monomer (a2), those described in Japanese Patent No. 3648553, Japanese Patent Publication No. 2003-165883, Japanese Patent Publication No. 2005-75982, and Japanese Patent Publication No. 2005-95759 can be further used. The water-soluble ethylenically unsaturated monomer (a1) and the hydrolyzable monomer (a2) may be used alone or as a mixture of two or more kinds of monomers, respectively.

In addition to the water-soluble ethylenically unsaturated monomer (a1) and the hydrolyzable monomer (a2), another vinyl monomer (a3) that is copolymerizable with these monomers can be used. As the copolymerizable other vinyl monomer (a3), hydrophobic vinyl monomers and the like can be used, but it is not limited to them. As the other vinyl monomer (a3), the following vinyl monomers (i) to (iii) and the like are used.

(i) Aromatic ethylenically unsaturated monomers having 8 to 30 carbon atoms;
Styrenes such as styrene, alpha-methylstyrene, vinyltoluene, and hydroxystyrene; vinylnaphthalene; and halogen substitutions of styrene such as dichlorostyrene.
(ii) Aliphatic ethylenically unsaturated monomers having 2 to 20 carbon atoms;
Alkenes such as ethylene, propylene, butene, isobutylene, pentene, heptene, diisobutylene, octene, dodecene, and octadecene; and alkadienes such as butadiene, and isoprene.
(iii) Alicyclic ethylenically unsaturated monomers having 5 to 15 carbon atoms;
Mono-ethylenically unsaturated monomers such as pinene, limonene, and indene; and polyethylenic vinyl-polymerizable monomers such as cyclopentadiene, bicyclopentadiene, and ethylidene norbornene.

As the other vinyl monomer (a3), those described in Japanese Patent No. 3648553, Japanese Publication No. 2003-165883, Japanese Patent Publication No. 2005-75982, and Japanese Patent Publication No. 2005-95759 can be further used.

From the aspect of providing the crosslinked polymer mainly composed of acrylic acid, as the water-soluble ethylenically unsaturated monomer (a1) and/or the hydrolyzable monomer (a2) producing the water-soluble ethylenically unsaturated monomer (a1) by hydrolysis, acrylic acid or a salt of acrylic acid (a1), or a hydrolyzable monomer (a2) producing acrylic acid or the salt of acrylic acid is preferable. The content of acrylic acid or the salt of acrylic acid (a1), or the hydrolyzable monomer (a2) producing acrylic acid or the salt of acrylic acid in the unsaturated monomer composition constituting the crosslinked polymer is preferably 90 mass% or more, more preferably 95 mass% or more, and is preferably 99 mass% or less, more preferably 97 mass% or less.

Examples of the internal crosslinking agent (b) can include an internal crosslinking agent (b1) having two or more ethylenically unsaturated groups, an internal crosslinking agent (b2) having: at least one functional group that can react with a water-soluble substituent of the water-soluble ethylenically unsaturated monomer (a1) and/or a water-soluble substituent produced by hydrolysis of the hydrolyzable monomer (a2); and at least one ethylenically unsaturated group, and an internal crosslinking agent (b3) having at least two functional groups that can react with a water-soluble substituent of the water-soluble ethylenically unsaturated monomer (a1) and/or a water-soluble substituent produced by hydrolysis of the hydrolyzable monomer (a2).

Examples of the internal crosslinking agent (b1) having two or more ethylenically unsaturated groups include bis(meth)acrylamides having 8 to 12 carbon atoms, poly(meth)acrylates of polyols having 2 to 10 carbon atoms, polyallylamines having 2 to 10 carbon atoms, and poly(meth)allyl ethers of polyols having 2 to 10 carbon atoms. Specific examples of them include N,N'-methylene bis(meth)acrylamide, ethylene glycol di(meth)acrylate, poly (polymerization degree of 2 to 5) ethylene glycol di(meth)acrylate, propylene glycol di(meth)acrylate, glycerol (di or tri)acrylate, trimethylol propane triacrylate, diallylamine, triallylamine, triallylcyanurate, triallylisocyanurate, tetraallyloxyethane, pentaerythritol diallyl ether, pentaerythritol triallyl ether, pentaerythritol tetraallyl ether, and diglycerin di(meth)acrylate.

Examples of the internal crosslinking agent (b2) having at least one functional group that can react with a water-soluble substituent of the water-soluble ethylenically unsaturated monomer (a1) and/or a water-soluble substituent produced by hydrolysis of the hydrolyzable monomer (a2) and at least one ethylenically unsaturated group include ethylenically unsaturated compounds having 6 to 8 carbon atoms and an epoxy group, ethylenically unsaturated compounds having 4 to 8 carbon atoms and a hydroxyl group, and ethylenically unsaturated compounds having 4 to 8 carbon atoms and an isocyanato group. Specific examples of them include glycidyl (meth)acrylate, N-methylol (meth)acrylamide, hydroxyethyl (meth)acrylate, and isocyanato ethyl (meth)acrylate.

Examples of the internal crosslinking agent (b3) having at least two functional groups that can react with a water-soluble substituent of the water-soluble ethylenically unsaturated monomer (a1) and/or a water-soluble substituent produced by hydrolysis of the hydrolyzable monomer (a2) can include polyhydric alcohols, polyvalent glycidyls, polyvalent amines, polyvalent aziridines, and polyvalent isocyanates. Examples of polyvalent glycidyl compounds include ethylene glycol diglycidyl ether and glycerin diglycidyl ether. Examples of polyvalent amine compounds include ethylenediamine, diethylenetriamine, triethylenetetramine, tetraethylenepentamine, pentaethylenehexamine, and polyethyleneimine. Examples of polyvalent aziridine compounds include Chemitite PZ-33 {2,2-bishydroxymethylbutanol-tris(3-(1-aziridinyl)propionate)}, Chemitite HZ-22 {1,6-hexamethylenediethyleneurea}, and Chemitite DZ-22 {diphenylmethane-bis-4,4'-N,N'-diethyleneurea}, available from Nippon Shokubai Co., Ltd. Examples of polyvalent polyisocyanate compounds include 2,4-tolylene diisocyanate and hexamethylene diisocyanate. These internal crosslinking agents may be used singly or two or more of them may be used in combination.

As the internal crosslinking agent (b), in light of absorbing performance (in particular, an absorption amount, an absorption speed, etc.), the internal crosslinking agent (b1) having two or more ethylenically unsaturated groups is preferred, poly(meth)allyl ethers of polyols having 2 to 10 carbon atoms are more preferred, triallylcyanurate, triallylisocyanurate, tetraallyloxyethane, or pentaerythritol triallyl ether is further preferred, and pentaerythritol triallyl ether is most preferred.

As the internal crosslinking agent (b), those described in Japanese Patent No. 3648553, Japanese Patent Publication No. 2003-165883, Japanese Patent Publication No. 2005-75982, and Japanese Patent Publication No. 2005-95759 can be further used.

As the method for polymerizing the crosslinked polymer (A), a conventionally known method and the like can be used, and a solution polymerization method, an emulsion polymerization method, a suspension polymerization method, and a reversed-phase suspension polymerization method can be used. In addition, a polymerization liquid at the polymerization may be in the form of a thin film, mist, or the like. As the method for controlling the polymerization, an adiabatic polymerization method, a temperature-controlled polymerization method, an isothermal polymerization method, and the like can be used. As the polymerization method, the solution polymerization method is preferred, and an aqueous solution polymerization method is more preferred since an organic solvent and the like are not used and it is advantageous in terms of production cost.

A water-containing gel {consisting of the crosslinked polymer and water} obtained by the polymerization can be chopped where necessary. The size (largest diameter) of the chopped gel is preferably from 50 micrometers to 10 cm, more preferably from 100 micrometers to 2 cm, and even more preferably from 1 mm to 1 cm. If the size falls within this range, dryability during a drying process becomes further favorable.

The chopping can be conducted by a known method, and can be conducted, for example, by using a conventional chopping apparatus such as a Bexmill, a rubber chopper, a Pharma Mill, a mincing machine, an impact type mill, and a roll type mill.

When a solvent (an organic solvent, water, etc.) is used for the polymerization, it is preferred to remove the solvent by distillation after the polymerization. When the solvent contains water, the water content (mass %) with respect to the mass (100 mass %) of the crosslinked polymer after the removal by distillation is preferably from 0 mass % to 20 mass %, more preferably from 1 mass % to 10 mass %, even more preferably from 2 mass % to 9 mass %, and most preferably from 3 mass % to 8 mass %. When the water content (% by mass) falls within the above range, the absorbing performance and the breakability of the water-absorbent resin powder after drying become further favorable.

It is noted that the content of the organic solvent and the water content are obtained based on a decrease in the mass of a measurement sample from before heating to after heating by an infrared moisture measuring instrument {JE400 manufactured by Kett Electric Laboratory or the like: 120 plus or minus 5 degrees centigrade, 30 minutes, an atmospheric humidity before heating of 50 plus or minus 10% RH, lamp specifications of 100 V and 40 W}.

As the method for removing the solvent (including water) by distillation, a method in which removal by distillation (drying) is conducted by hot air at a temperature in a range from 80 degrees centigrade to 230 degrees centigrade, a thin film drying method with a drum dryer or the like heated at the temperature in a range from 100 degrees centigrade to 230 degrees centigrade, a (heating) reduced-pressure drying method, a freeze-drying method, a drying method with infrared rays, decantation, filtration, and the like can be used.

The crosslinked polymer (A) can be pulverized after being dried. The pulverizing method is not particularly limited, and, for example, an ordinary pulverizing apparatus such as a hammer type pulverizer, an impact type pulverizer, a roll type pulverizer, and a jet streaming type pulverizer can be used. The particle size of the pulverized crosslinked polymer (A) can be adjusted by sieving or the like where necessary.

The weight average particle size (micrometer) of the crosslinked polymer (A) that is sieved where necessary is preferably from 100 micrometers to 800 micrometers, more preferably from 200 micrometers to 700 micrometers, even more preferably from 250 micrometers to 600 micrometers, particularly preferably from 300 micrometers to 500 micrometers, and most preferably from 350 micrometers to 450 micrometers. When the weight average particle size (micrometer) of the crosslinked polymer (A) falls within the above range, the absorbing performance becomes further favorable.

It is noted that the weight average particle size is measured with a ro-tap test sieve shaker and standard sieves (JIS Z8801-1: 2006) according to the method described in Perry's Chemical Engineers Handbook, Sixth Edition (The McGraw-Hill Companies, 1984, Page 21). In other words, as JIS standard sieves, for example, sieves of 1000 micrometers, 850 micrometers, 710 micrometers, 500 micrometers, 425 micrometers, 355 micrometers, 250 micrometers, 150 micrometers, 125 micrometers, 75 micrometers, and 45 micrometers, and a tray are combined in order from above. About 50 g of a measurement particle is placed into the uppermost sieve, and shaken with the ro-tap test sieve shaker for 5 minutes. The weights of the measurement particles on each sieve and the tray are measured, and the weight fraction of the particles on each sieve is obtained with the total weight regarded as 100% by weight. The values are plotted in a log probability paper {the horizontal axis is used for the opening of the sieve (particle size) and the vertical axis is used for the weight fraction}, then a line is drawn so as to connect each point, and a particle size corresponding to 50% by mass of the mass fraction is obtained and regarded as a weight average particle size.

The surface of the crosslinked polymer (A) may be treated with the surface modifier (B). Examples of the surface modifier (B) include polyvalent metal compounds such as aluminum sulfate, potassium alum, ammonium alum, sodium alum, (poly) aluminum chloride, and hydrates thereof; polycation compounds such as polyethyleneimine, polyvinylamine, and polyallylamine; inorganic fine particles; a surface modifier (B1) containing a hydrocarbon group; a surface modifier (B2) containing a hydrocarbon group having a fluorine atom; and a surface modifier (B3) having a polysiloxane structure.

Examples of the inorganic fine particles include oxides such as silicon oxide (silica), aluminum oxide (alumina), iron oxide, titanium oxide, magnesium oxide, and zirconium oxide, carbides such as silicon carbide and aluminum carbide, nitrides such as titanium nitride, and complexes thereof (e.g., zeolite, talc, etc.). Among them, oxides are preferred, and silicon oxide is further preferred. The volume average particle size of the inorganic fine particles is preferably from 10 nm to 5000 nm, more preferably from 30 nm to 1000 nm, even more preferably from 50 nm to 750 nm, and most preferably from 90 nm to 500 nm. It is noted that the volume average particle size is measured in a solvent by a dynamic light scattering method. Specifically, the volume average particle size is measured in cyclohexane as a solvent at a temperature of 25 degrees centigrade by using the nano track particle size distribution measuring instrument UPA-EX150 (light source: He-Ne laser) manufactured by Nikkiso Co., Ltd.

Examples of the surface modifier (B 1) containing a hydrocarbon group include polyolefin resins, polyolefin resin derivatives, polystyrene resins, polystyrene resin derivatives, waxes, long-chain fatty acid esters, long-chain fatty acids and salts thereof, long-chain aliphatic alcohols, and mixtures of two or more of them.

Examples of the surface modifier (B2) containing a hydrocarbon group having a fluorine atom include perfluoroalkanes, perfluoroalkenes, perfluoroaryls, perfluoroalkyl ethers, perfluoroalkylcarboxylic acids or salts thereof, perfluoroalkyl alcohols, and mixtures of two or more of them.

Examples of the surface modifier (B3) having a polysiloxane structure include polydimethylsiloxane; polyether-modified polysiloxanes such as polyoxyethylene-modified polysiloxane and poly(oxyethylene/oxypropylene)-modified polysiloxane; carboxy-modified polysiloxanes; epoxy-modified polysiloxanes; amino-modified polysiloxanes; alkoxy-modified polysiloxanes; and mixtures thereof.

As the surface modifier (B), in light of absorption properties, the surface modifier (B3) having a polysiloxane structure and inorganic fine particles are preferred, and amino-modified polysiloxanes, carboxy-modified polysiloxanes, and silica are more preferred.

The method for treating the crosslinked polymer (A) with the surface modifier (B) is not particularly limited, as long as treatment is conducted such that the surface modifier (B) is present on the surface of the crosslinked polymer (A). However, from the standpoint that the amount of the surface modifier (B) on the surface is controlled, it is preferred that the surface modifier (B) is mixed with a dried product of the crosslinked polymer (A), not with a water-containing gel of the crosslinked polymer (A) or a polymerization liquid that is prior to polymerization of the crosslinked polymer (A). It is noted that it is preferred that the mixing is uniformly conducted.

The shape of the water-absorbent resin powder is not particularly limited, and examples thereof include an indefinite crushed shape, a scale shape, a pearl shape, a rice grain shape, or the like. Among them, the indefinite crushed shape is preferred from the standpoint that the powder in such a shape can be well entangled with fibrous materials in applications such as a disposable diaper and there is little possibility of the powder falling off from the fibrous materials.

The water-absorbent resin powder can be subjected to surface crosslinking where necessary. As a crosslinking agent for conducting the surface crosslinking (a surface crosslinking agent), the same ones as the internal crosslinking agent (b) can be used. In light of absorption performance and the like of the water-absorbent resin powder, the surface crosslinking agent is preferably the crosslinking agent (b3) having at least two functional groups that can react with a water-soluble substituent of the water-soluble ethylenically unsaturated monomer (a1) and/or a water-soluble substituent produced by hydrolysis of the hydrolyzable monomer (a2), more preferably a polyvalent glycidyl, even more preferably ethylene glycol diglycidyl ether and glycerin diglycidyl ether, and most preferably ethylene glycol diglycidyl ether.

In the case of conducting the surface crosslinking, the content (mass %) of the surface crosslinking agent with respect to the total mass (100 mass %) of the water-soluble ethylenically unsaturated monomer (a1) and/or the hydrolyzable monomer (a2), the internal crosslinking agent (b), and the other vinyl monomer (a3) used where necessary is preferably from 0.001 mass % to 7 mass %, more preferably from 0.002 mass % to 5 mass %, and even more preferably 0.003 mass % to 4 mass %. In other words, in this case, the upper limit of the content of the surface crosslinking agent based on the total mass of (a1) and/or (a2), (b), and (a3) is preferably 7 mass %, more preferably 5 mass %, and even more preferably 4 mass %. Similarly, the lower limit is preferably 0.001 mass %, more preferably 0.002 mass %, and even more preferably 0.003 mass %. If the content of the surface crosslinking agent falls within the above range, the absorption performance becomes further favorable. The surface crosslinking can be achieved by, for example, a method of spraying an aqueous solution containing the surface crosslinking agent to the water-absorbent resin powder or impregnating the water-absorbent resin powder with the aqueous solution containing the surface crosslinking agent, followed by heating treatment (100 to 200 degrees centigrade) on the water-absorbent resin powder.

The water-absorbent resin powder can contain additives such as an antiseptic, a fungicide, an antibacterial, an antioxidant, a ultraviolet absorber, a coloring agent, a perfuming agent, a deodorizer, an inorganic powder, and an organic fibrous material. Examples of such additives include those exemplified in Japanese Patent Publication No. 2003-225565 and Japanese Patent Publication No. 2006-131767. When these additives are contained, the content (mass %) of the additives with respect to the crosslinked polymer (A) (100 mass %) is preferably from 0.001 mass % to 10 mass %, more preferably from 0.01 mass % to 5 mass %, even more preferably from 0.05 mass % to 1 mass %, and most preferably from 0.1 mass % to 0.5 mass %.

Diffusibility improvement material

Next, the diffusibility improvement material used in the present invention will be described. The diffusibility improvement material is a material for improving the diffusibility of the body fluid or the like in the absorbent body, especially a material for improving the diffusibility under the load. The diffusibility improvement material preferably has an under-load liquid passing rate of 15 seconds or less, more preferably 12 seconds or less, even more preferably 10 seconds or less, further more preferably 8 seconds or less. If the diffusibility improvement material has an under-load liquid passing rate of 15 seconds or less, the diffusion rate of the body fluid or the like into the absorbent body becomes good. It is noted that the method for measuring the under-load liquid passing rate of the diffusibility improvement material is described later.

The form of the diffusibility improvement material is a granular form. According to the invention, the granular diffusibility improvement material includes resin particles such as polypropylene particles, polystyrene particles, and ABS (acrylonitrile -butadiene-styrene copolymer) resin particles. It should be noted that these resin particles do not substantially have any water absorptivity.

The particle diameter of the granular diffusibility improvement material is 0.05 mm or more, preferably 0.1 mm or more, further preferably 0.5 mm or more, and particularly preferably 1.0 mm or more, and is 10 mm or less, preferably 7 mm or less, and further preferably 6 mm or less. If the particle diameter is within the above described range, the diffusibility of body fluid or the like becomes excellent.

Examples of a sheet-like diffusibility improvement material include a three-dimensional structural body having a void. Examples of such three-dimensional structural body include: natural products such as a dried sponge-gourd; a three-dimensional interlaced body of a resin fiber (e.g., BREATHAIR (Registered trademark) manufactured by Toyobo Co., Ltd.); and a three-dimensional stereo knitted goods of a resin fiber (e.g., Fusion (Registered trademark) manufactured by Asahi Kasei Fibers Corp.). It should be noted that since nonwoven fabrics and the like conventionally used as an absorbent article have thin fibers, void included therein will be crushed when a load is applied. This results in an under-load liquid passing rate larger than 15 seconds, which does not qualify as a diffusibility improvement material that is to be used in the present invention.

Configuration of absorbent body

The configuration of the absorbent body of the present invention is not particularly limited as long as there is a layer having formed at least partially thereon the diffusion region where a diffusibility improvement material having an under-load liquid passing rate of 15 seconds or less is disposed. Other than the diffusibility improvement material, the diffusion region may also include additives such as a preservative, an antifungal agent, an antibacterial agent, an antioxidant, an ultraviolet ray absorbing agent, a coloring agent, a fragrance material, a deodorizer, an inorganic powder, and an organic fibrous material, unless the advantageous effect of the present invention deteriorates. In this case, the under-load liquid passing rate of the diffusibility improvement material in a state including the additive is preferably 10 seconds or less. It should be noted that the water absorbent resin powder is not disposed in the diffusion region.

Examples of the layer having formed at least partially thereon the diffusion region include: a diffusion layer only having the diffusion region; and a diffusion-and-water absorption layer having the diffusion region and a water absorption region where the water absorbent resin powder is disposed.

The diffusion layer mainly has a function of diffusing body fluid or the like. When the diffusion layer is formed from a sheet-like diffusibility improvement material, its thickness is preferably 0.5 mm or more, more preferably 2 mm or more, and further preferably 2.5 mm or more, and is preferably 15 mm or less, more preferably 10 mm or less, and further preferably 7 mm or less. When the thickness of the diffusion layer formed from the sheet-like diffusibility improvement material is within the above described range, the diffusibility can be improved without impairing the texture. When the diffusion layer is formed from a particulate diffusibility improvement material, its thickness is preferably 0.05 mm or more, more preferably 0.1 mm or more, and further preferably 0.15 mm or more, and is preferably 10 mm or less, more preferably 7 mm or less, and further preferably 6 mm or less. When the thickness of the diffusion layer formed from the particulate diffusibility improvement material is within the above described range, the diffusibility can be improved without impairing the texture.

The diffusion-and-water absorption layer has the functions of diffusing body fluid or the like in the diffusion region in the thickness direction and the planar direction of the layer, and absorbing body fluid in the water absorption region. In the diffusion-and-water absorption layer, the size and shape of the diffusion region is not particularly limited. Examples of the planar view shape of the diffusion region on the surface of the diffusion-and-water absorption layer include a circular shape, an elliptical shape, a polygonal shape (e.g., rectangular shape, triangular shape), a polygonal shape having rounded corners (the shape of a polygon whose vertices are rounded), and a slit shape (e.g., linear slit, wavy slit). Furthermore, the number of the diffusion region is not particularly limited, and a single or multiple (two or more) diffusion regions may be formed.

When only a single diffusion region is formed, the diffusion region is preferably formed so as to span over the central line of the absorbent body in the width direction. Furthermore, when the planar view shape of the diffusion region is an elliptical shape or a rectangular shape, the longitudinal axis direction or long side direction thereof is preferably substantially parallel with the longitudinal direction of the diffusion-and-water absorption layer. When the diffusion region has a slit shape, the longitudinal direction of the slit is preferably substantially parallel with the longitudinal direction of the diffusion-and-water absorption layer.

When multiple diffusion regions are formed, arrangements thereof are not particularly limited. Examples of the arrangements of the diffusion region whose planar view shape is a circular shape, an elliptical shape, a polygonal shape (e.g., rectangular shape), or a polygonal shape having rounded corners include an alternate pattern, and a grid pattern. Examples of the arrangements of the slit shaped diffusion regions include a mode in which the slit shaped diffusion regions are arranged parallel to each other, and a mode in which the slit shaped diffusion regions are arranged so as to intersect with each other.

It should be noted that, when the planar view shape of the diffusion region is a circular shape, an elliptical shape, a polygonal shape, (e.g., a rectangular shape), or a polygonal shape having rounded corners, it is also preferable to form the diffusion region so as to match the position where body fluid or the like is excreted. By having such a configuration, dryness of a specific site can be improved. One such embodiment includes, for example, forming the diffusion region from an approximately center part of the diffusion-and-water absorption layer in a longitudinal direction, toward one side thereof in the longitudinal direction. By forming the diffusion region in such manner, the dryness of a portion which is in contact with the hip of a wearer can be improved.

In the diffusion-and-water absorption layer, the area ratio of the diffusion region with respect to the surface of the diffusion-and-water absorption layer is preferably 5% or more, more preferably 15% or more, and even more preferably 20% or more, and is preferably 70% or less, more preferably 50% or less, and further preferably 40% or less. If the area ratio is 5% or more, the absorption rate of body fluid or the like is further improved, and if the area ratio is 70% or less, further excellent dryness is obtained after body fluid or the like is absorbed.

Similar to the diffusion layer, the thickness of the portion of the diffusion region of the diffusion-and-water absorption layer can be appropriately adjusted depending on the material forming the diffusion region. When a sheet-like diffusibility improvement material and a particulate diffusibility improvement material are used, the suitably range for the thickness of the diffusion region is similar to that for the diffusion layer.

The water absorption region is formed from a water absorbent material such as a water absorbent resin powder or a water absorbent fiber. Examples of the water absorbent fiber include pulp fibers, cellulose fibers, rayon, and acetate fibers. The water absorption region may include a fiber base-material in addition to a water absorbent resin powder. Examples of the fiber base-material include thermal bonding fibers and the like. Thermal bonding fibers are used to enhance shape retention. Specific examples of the thermal bonding fibers include polyolefin fibers such as polyethylene and polypropylene, polyester fibers, and conjugate fibers.

When the planar view shape of the diffusion region is a circular shape, an elliptical shape, a polygonal shape (e.g., rectangular shape), or a polygonal shape having rounded corners, the water absorption region is preferably formed so as to surround the diffusion region.

The diffusion region and the water absorption region may be formed by fixing the diffusibility improvement material, the water absorbent material, and the like to a paper sheet such as tissue paper, or a liquid-permeable nonwoven fabric sheet; or by wrapping the diffusibility improvement material, the water absorbent material, and the like with a paper sheet such as tissue paper, or a liquid-permeable nonwoven fabric sheet to mold the wrapped product into a desired shape.

Examples of the configuration of the absorbent body of the present invention include: (1) a configuration including at least one layer of the diffusion-and-water absorption layer; and (2) a configuration including at least one layer of the diffusion layer, and the water absorption layer.

The configuration of (1) described above may also include a water absorption layer in addition to the diffusion-and-water absorption layer. By having the water absorption layer, water retention amount of the absorbent body is further improved. Furthermore, there may be multiple diffusion-and-water absorption layers and multiple water absorption layers, respectively. It should be noted that, when the diffusion-and-water absorption layer and the water absorption layer are included, the diffusion-and-water absorption layer is preferably disposed on a side closest to the skin surface. By disposing the diffusion-and-water absorption layer on a side closest to the skin surface, even when gel-blocking has occurred in the water absorption region of the diffusion-and-water absorption layer, the body fluid or the like is taken into the lower water absorption layer via the diffusion region. As a result, the performance of the water absorbent resin powder disposed below the diffusion-and-water absorption layer is also sufficiently exerted.

When the water absorption layer is to be laminated, the shape of the water absorption layer may be substantially identical to the shape of the diffusion-and-water absorption layer, and its size may be larger or smaller than that of the diffusion-and-water absorption layer. From a standpoint of enhancing absorption performance of the absorbent body, the water absorption layer is preferably disposed below the diffusion region formed in the diffusion-and-water absorption layer.

In the configuration of (1) described above, the water absorbent resin powder (S1) used in the water absorption layer preferably satisfies the following requirements.

The absorption ratio of the water absorbent resin powder (S1) is preferably 40 g/g or more, more preferably 45 g/g or more, and further preferably 50 g/g or more, and is preferably 70 g/g or less, more preferably 65 g/g or less, and further preferably 60 g/g or less. When the absorption ratio is 40 g/g or more, absorptive capacity can be maintained at a predetermined level with a small amount of the water absorbent resin powder, and it becomes easy to manufacture a thin absorbent body. From a standpoint of preventing liquid leakage, an absorption ratio is preferably as large as possible. However, when the absorption ratio is 70 g/g or less, the stability of the water absorbent resin powder against urine is improved.

The water retention amount of the water absorbent resin powder (S1) is preferably 30 g/g or more, more preferably 35 g/g or more, and further preferably 37 g/g or more, and preferably 50 g/g or less, more preferably 47 g/g or less, and further preferably 45 g/g or less. When the water retention amount is 30 g/g or more, body fluid retention volume can be maintained at a predetermined level with a small amount of the water absorbent resin powder, and it becomes easy to manufacture a thin absorbent body. From a standpoint of preventing liquid leakage, a water retention amount is preferably as large as possible. However, when the water retention amount is 50 g/g or less, the stability of the water absorbent resin powder against urine is improved.

In the configuration of (2) described above, there may be multiple diffusion layers and multiple water absorption layers. It should be noted that, of the diffusion layer and the water absorption layer, the diffusion layer is preferably disposed on a side closest to the skin surface. By disposing the diffusion layer in such manner, the body fluid or the like is absorbed by the lower water absorption layer while being diffused in a planar direction in the diffusion layer. Therefore, even when gel-blocking occurs partially at the water absorption region, since the body fluid or the like is easily transferred to a portion where gel-blocking has not occurred via the diffusion layer, the water absorption rate is improved.

In the configuration of (2) described above, the water absorbent resin powder (S2) used in the water absorption layer preferably satisfies the following requirements.

The absorption ratio of the water absorbent resin powder (S2) is preferably 50 g/g or more, more preferably 53 g/g or more, and further preferably 55 g/g or more, and is preferably 70 g/g or less, more preferably 65 g/g or less, and further preferably 60 g/g or less. When the absorption ratio is 50 g/g or more, the absorptive capacity can be maintained at a predetermined level with a small amount of the water absorbent resin powder, and it becomes easy to manufacture a thin absorbent body. From a standpoint of preventing liquid leakage, an absorption ratio is preferably as large as possible. However, when the absorption ratio is 70 g/g or less, the stability of the water absorbent resin powder against urine is improved.

The water retention amount of the water absorbent resin powder (S2) is preferably 25 g/g or more, more preferably 27 g/g or more, further preferably 30 g/g or more, and particularly preferably 40 g/g or more, and is preferably 60 g/g or less, more preferably 57 g/g or less, and further preferably 55 g/g or less. When the water retention amount is 25 g/g or more, body fluid retention volume can be maintained at a predetermined level with a small amount of the water absorbent resin powder, and it becomes easy to manufacture a thin absorbent body. From a standpoint of preventing liquid leakage, a water retention amount is preferably as large as possible. However, when the water retention amount is 60 g/g or less, stability of the water absorbent resin powder against urine is improved.

The absorption rate of the water absorbent resin powder (S2) determined by a vortex method is preferably 5 seconds or more, more preferably 7 seconds or more, and further preferably 10 seconds or more, and is preferably 60 seconds or less, more preferably 40 seconds or less, further preferably 20 seconds or less, and particularly preferably 15 seconds or less. Although an absorption rate is preferably as small as possible, when the absorption rate is 5 seconds or more, the stability of the water absorbent resin powder against urine, particularly its under-load stability, becomes further excellent. When the absorption rate is 60 seconds or less, even when a large amount of body fluid is excreted at a high excretion rate at once, the body fluid can be sufficiently absorbed and therefore liquid leakage is further suppressed.

In the absorbent body of the present invention, the shapes of the diffusion layer, the diffusion-and-water absorption layer, and the water absorption layer are not particularly limited, and examples thereof include a rectangular shape, an hourglass shape, a gourd shape, a battledore shape, and the like. Furthermore, in addition to the diffusion layer, the diffusion-and-water absorption layer, and the water absorption layer; the absorbent body may include a nonwoven fabric layer, and an adhesive layer for fixing the diffusibility improvement material and the water absorbent resin powder.

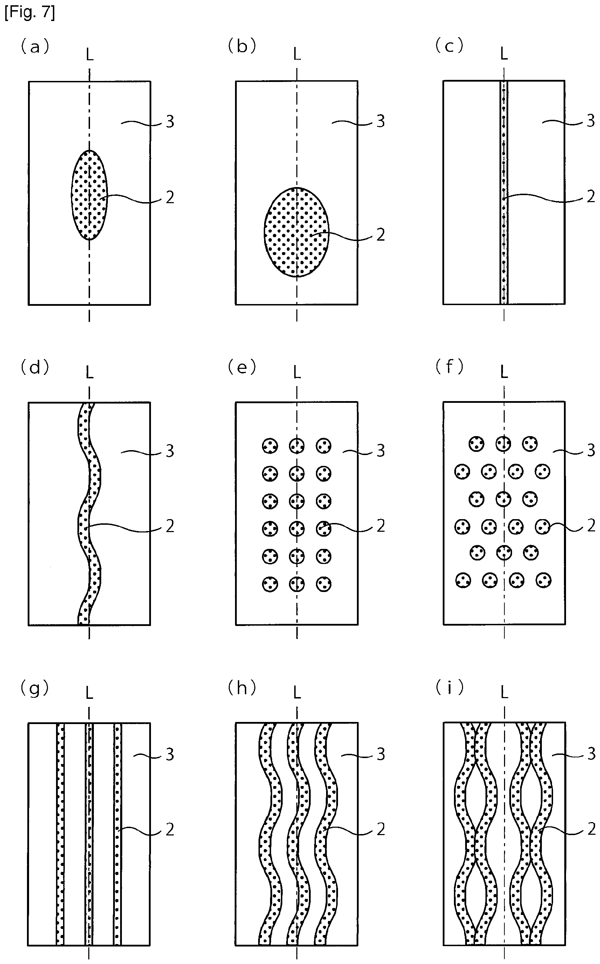

In the following, although description of a preferable embodiment of the absorbent body of the present invention will be provided with reference to the drawings, the present invention is not limited to the mode that has been diagrammatically represented. Specific examples of the mode of the diffusion-and-water absorption layer in the configuration of (1) described above will be described with reference to FIG. 7. FIG. 7 is a plan view of a diffusion-and-water absorption layer from the skin surface side, reference character "2" indicates a diffusion region, reference character "3" indicates a water absorption region, and a dot and dash line "L" indicates the central line of the diffusion-and-water absorption layer in the width direction.

Examples of the mode of the diffusion-and-water absorption layer include: a mode ((a) of FIG. 7) in which a single elliptical shaped diffusion region 2 is formed at a substantially central portion of the diffusion-and-water absorption layer in the longitudinal direction, such that a longitudinal axis direction of the diffusion region 2 and the longitudinal direction of the diffusion-and-water absorption layer are substantially parallel; a mode ((b) of FIG. 7) in which a single elliptical shaped diffusion region 2 is formed toward one side in the longitudinal direction (e.g., hip side of a wearer) from an approximately center part of a diffusion-and-water absorption layer 4 in the longitudinal direction, such that the longitudinal axis direction of the diffusion region 2 and the longitudinal direction of the diffusion-and-water absorption layer are substantially parallel; a mode ((c) of FIG. 7) in which a single linear-slit shaped diffusion region 2 is formed; a mode ((d) of FIG. 7) in which a single wavy-slit shaped diffusion region 2 is formed; a mode ((e) of FIG. 7) in which a plurality of circular shaped diffusion regions 2 are formed in a grid-pattern arrangement; a mode ((f) of FIG. 7) in which a plurality of circular shaped diffusion regions 2 are formed in an alternate arrangement; a mode ((g) of FIG. 7) in which a plurality of linear slit shaped diffusion regions 2 are formed parallel to each other; a mode ((h) of FIG. 7) in which a plurality of wavy slit shaped diffusion regions 2 are formed parallel to each other; and a mode ((i) of FIG. 7) in which wavy slit shaped diffusion regions 2 are formed so as to intersect with each other. It should be noted that, in (a) to (d) of FIG. 7, the diffusion regions 2 is formed so as to span over a central line L of the diffusion-and-water absorption layer in the width direction.

Examples of a preferable embodiment of the absorbent body of the present invention include: a mode with a single diffusion-and-water absorption layer (Embodiment 1); a mode with at least two or more layers including a diffusion-and-water absorption layer having formed thereon a slit shaped diffusion region, and a water absorption layer only having a water absorption region below the diffusion-and-water absorption layer (Embodiment 2); and a mode with at least two or more layers including a diffusion layer only having a diffusion region, and a water absorption layer only having a water absorption region below the diffusion layer (Embodiment 3).

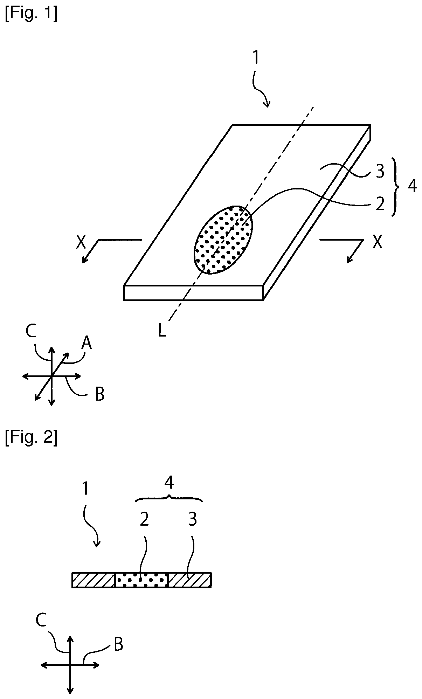

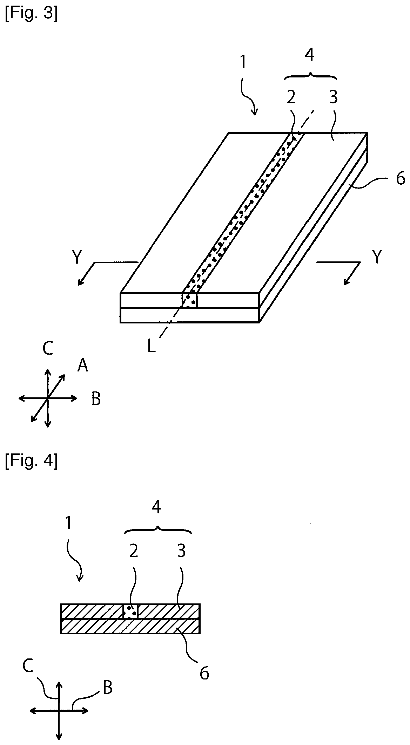

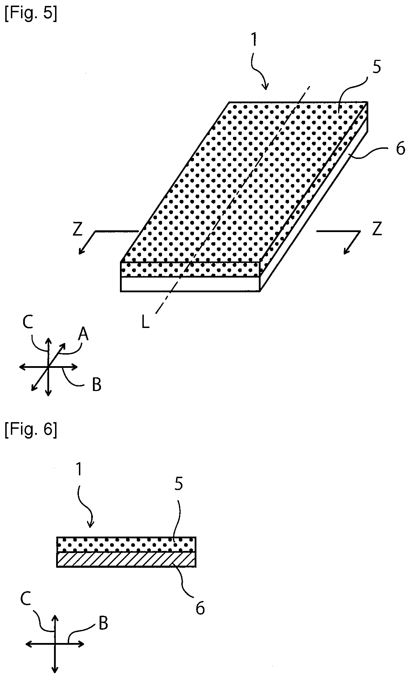

FIGS. 1 and 2 are schematic diagrams showing Embodiment 1 of the absorbent body of the present invention. FIG. 1 is a schematic perspective view from a skin surface side, and FIG. 2 is a cross sectional view along line X-X in FIG. 1. FIGS. 3 and 4 are schematic diagrams showing Embodiment 2 of the absorbent body of the present invention. FIG. 3 is a schematic perspective view from a skin surface side, and FIG. 4 is a cross sectional view along line Y-Y in FIG. 3. FIGS. 5 and 6 are schematic diagrams showing Embodiment 3 of the absorbent body of the present invention. FIG. 5 is a schematic perspective view from a skin surface side, and FIG. 6 is a cross sectional view along line Z-Z in FIG. 5. It should be noted that, in FIGS. 1 to 6, with respect to C direction on the paper surface, the upper side is the skin surface side, whereas the lower side is the external surface side.

Embodiment 1

Embodiment 1 is an absorbent body 1 composed of a single diffusion-and-water absorption layer 4. The absorbent body 1 consists of the diffusion-and-water absorption layer 4 including a diffusion region 2 where a diffusibility improvement material is disposed and a water absorption region 3 where a water absorbent resin powder is disposed. Since the diffusion-and-water absorption layer 4 has the water absorption region 3 surrounding the diffusion region 2, body fluid or the like taken into the diffusion region 2 is immediately absorbed by the adjacent water absorption region 3. As a result, absorption rate of the body fluid or the like is further improved. In Embodiment 1, since the diffusion-and-water absorption layer 4 is formed from a single layer, reduction in thickness thereof is possible. Therefore, the absorbent body of Embodiment 1 is suitable as an absorbent article for light incontinence.

In the absorbent body 1 shown in FIG. 1, the diffusion-and-water absorption layer 4 has a rectangular shape with a longitudinal direction A and a short direction B. Furthermore, on the diffusion-and-water absorption layer 4, the diffusion region 2 whose planar view shape is an elliptical shape is singly formed and the water absorption region 3 is formed so as to surround the circumference thereof. In addition, the diffusion region 2 is formed so as to span over the central line L of the absorbent body 1 (the diffusion-and-water absorption layer 4) in the width direction B, and also formed starting from an approximately center part of the absorbent body 1 in the longitudinal direction A, toward one side in the longitudinal direction.

In FIG. 1, although a rectangular shape is diagrammatically represented as the shape of the absorbent body 1, the shape of the absorbent body may be an hourglass shape, a gourd shape, a battledore shape, or the like. In FIG. 1, although the number of the diffusion region is one, the number of the diffusion regions may be two or more. Furthermore, although the planar view shape of the diffusion region is formed as an elliptical shape, the shape of the diffusion region is not limited thereto, and may be a circular shape, a polygonal shape, a polygonal shape having rounded corners, a slit shape, or the like.

Embodiment 2

Embodiment 2 is an absorbent body 1 composed of two layers, i.e., a diffusion-and-water absorption layer 4 having formed thereon a slit shaped diffusion region 2 and a water absorption layer 6 only having a water absorption region disposed below the diffusion-and-water absorption layer 4. By having such a configuration, body fluid or the like taken into the diffusion region 2 is immediately absorbed by adjacent water absorption regions 3, and body fluid or the like diffused in a planar direction within the diffusion region 2 is also absorbed by the water absorption layer 6 disposed below the diffusion region 2. As a result, even when body fluid or the like is discharged repeatedly, a fast absorption rate is obtained every time, and a large amount of the body fluid or the like can be absorbed. Therefore, the absorbent body of Embodiment 2 is suitable as an absorbent article required for repeated absorption.

In the absorbent body 1 shown in FIG. 3, the diffusion-and-water absorption layer 4 and the water absorption layer 6 both have a rectangular shape with a longitudinal direction A and a short direction B, and the planar shapes of the diffusion-and-water absorption layer 4 and the water absorption layer 6 are substantially identical. On the diffusion-and-water absorption layer 4, the linear slit shaped diffusion region 2 extending in the longitudinal direction of the absorbent body is singly formed, and the water absorption regions 3 are formed on both sides thereof in the width direction. The diffusion region 2 is formed so as to span over the central line L of the diffusion-and-water absorption layer 4 in the width direction B.

In FIG. 3, although a rectangular shape is diagrammatically represented as the shapes of the diffusion-and-water absorption layer 4 and the water absorption layer 6, the shapes may be an hourglass shape, a gourd shape, a battledore shape, or the like. In addition, the planar shape of the water absorption layer 6 may be smaller or larger than that of the diffusion-and-water absorption layer 4. In FIG. 3, although the diffusion-and-water absorption layer 4 and the water absorption layer 6 are each formed from a single layer, it is sufficient when at least a single water absorption layer is disposed below (external surface side) the diffusion-and-water absorption layer, and a nonwoven fabric layer or an adhesive layer may be inserted between the diffusion-and-water absorption layer and the water absorption layer. In addition, the diffusion-and-water absorption layer 4 and the water absorption layer 6 may each be formed from two or more layers. In FIG. 3, although a mode in which the linear slit shaped diffusion region 2 is singly formed in the diffusion-and-water absorption layer 4 is diagrammatically represented, the number of slits may be two or more. Furthermore, the shape of the slit may be a curved line such as a wavy line.

Embodiment 3

Embodiment 3 is an absorbent body 1 composed of two layers, i.e., a diffusion layer 5 only having a diffusion region and a water absorption layer 6 only having a water absorption region disposed below the diffusion layer. By having such a configuration, body fluid or the like taken into the absorbent body 1 is immediately diffused in a planar direction within the diffusion layer 5, and then absorbed by the water absorption layer 6 disposed below the diffusion layer 5. Thus, since the body fluid or the like is taken in from a broad range of the top surface of the water absorption layer 6, the absorption rate is further improved. Therefore, the absorbent body of Embodiment 3 is suitable as an absorbent article demanded to have a faster absorption rate. Furthermore, an absorbent article using the absorbent body of Embodiment 3 has a very large absorption rate and therefore has an excellent advantageous effect of preventing rashes.

In the absorbent body 1 shown in FIG. 5, the diffusion layer 5 and the water absorption layer 6 both have a rectangular shape with a longitudinal direction A and a short direction B, and the planar shapes of the diffusion layer 5 and the water absorption layer 6 are substantially identical. In FIG. 5, although a rectangular shape is diagrammatically represented as the shape of the absorbent body 1, the shape of the absorbent body may be an hourglass shape, a gourd shape, a battledore shape, or the like. Similarly, the shapes of the diffusion layer 5 and the water absorption layer 6 may be an hourglass shape, a gourd shape, a battledore shape, or the like. In addition, the planar shape of the water absorption layer 6 may be smaller or larger than that of the diffusion layer 5. In FIG. 5, although the diffusion layer 5 and the water absorption layer 6 are each formed from a single layer, it is sufficient when at least a single water absorption layer is disposed below (external surface side) the diffusion layer, and a nonwoven fabric layer or an adhesive layer may be inserted between the diffusion layer and the water absorption layer. In addition, the diffusion layer 5 and the water absorption layer 6 may each be formed from two or more layers.

Absorbent article

Next, description of a specific application example of the absorbent article of the present invention will be provided. Examples of the absorbent article of the present invention include absorbent articles used for absorbing body fluid discharged from the human body, such as an incontinence pad, a disposable diaper, a sanitary napkin, and a breast-milk pad.

When the absorbent article is an incontinence pad or a sanitary napkin, for example, the absorbent body is disposed between the top sheet and the back sheet. Examples of the shape of the incontinence pad or the sanitary napkin include a substantially rectangular shape, an hourglass shape, a gourd shape, and the like.

When the absorbent article is a disposable diaper, examples of the disposable diaper include: a tape-type disposable diaper that has one pair of securing members on right and left sides of a back portion or a front abdominal portion, and that forms, because of the securing member, a pants shape when being worn; and a pants-type disposable diaper having a waist opening and one pair of leg openings formed when a front abdominal portion and a back portion are joined together.

When the absorbent article is a disposable diaper, in the disposable diaper, for example, a laminated body including an inner sheet and an outer sheet may form a diaper main body including a front abdominal portion, a back portion, and a crotch portion located between those, and the absorbent body may be disposed on the crotch portion between the top sheet and the back sheet. Furthermore, the disposable diaper may include, for example, a laminated body having the absorbent body disposed between a top sheet and a back sheet, and the laminated body may include a front abdominal portion, a back portion, and a crotch portion located between those. It should be noted that, with regard to the front abdominal portion, the back portion, and the crotch portion, when the disposable diaper is worn, a portion placed on the abdominal side of a wearer is referred to as a front abdominal portion, a portion placed on the hip side of the wearer is referred to as a back portion, and a portion located between the front abdominal portion and the back portion and placed on the crotch of the wearer is referred to as a crotch portion. The inner sheet is preferably hydrophilic or water-repellent, and the outer sheet is preferably water-repellent.

The absorbent article preferably has rise flaps disposed along both side-edge portions of the absorbent body. For example, the rise flaps may be disposed on both side-edge portions of the top surface of the absorbent body in the width direction, or may be disposed on both outer sides of the absorbent body in the width direction. By providing the rise flaps, side-leakage of excrement such as urine can be prevented. The rise flaps may be formed by causing, to rise, inward edges of side sheets provided on both sides of the top sheet in the width direction. The rise flap and the side sheets are preferably water-repellent.

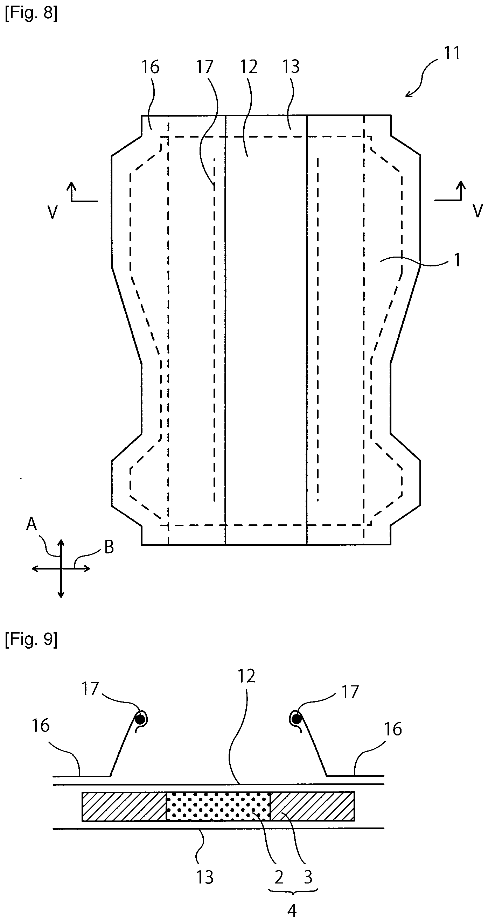

Next, using an incontinence pad as an example, the absorbent article of the present invention will be described with reference to FIGS. 8 and 9. FIG. 8 shows a plan view of an incontinence pad. FIG. 8 is a cross sectional view of an incontinence pad along V-V in FIG. 9. It should be noted that, in the figure, arrow B is defined as the width direction, and arrow A is defined as the longitudinal direction. Furthermore, a direction on the surface formed by arrows A and B is defined as a planar direction.

An incontinence pad 11 includes a liquid permeable top sheet 12, a liquid impermeable back sheet 13, and the absorbent body 1 disposed between those. FIG. 9 shows a cross sectional view of the incontinence pad 11. In this figure, although the absorbent body of Embodiment 1 described above is diagrammatically represented as the absorbent body 1, the configuration of the absorbent body is not limited thereto.

The top sheet 12 is disposed so as to face the skin at the crotch portion of the wearer, and allows passage of liquid excrement such as urine. Excrement such as urine that has passed through the top sheet 12 is taken into the absorbent body 1, diffused in a planar direction by the diffusion layer 5, and then moves to the water absorption layer 6 to be absorbed. Therefore, the incontinence pad 11 can immediately absorb the excrement, and the whole water absorbent resin powder included in the water absorption layer 6 can effectively contribute in the absorption of the excrement. The back sheet 13 prevents the excrement from leaking outside.

Side sheets 16 extending in the longitudinal direction A of the incontinence pad 11 are joined on both side edges of the top sheet 12 in the width direction B. The side sheets 16 are formed from a liquid impermeable plastic film, a water-repellent nonwoven fabric, or the like. The side sheets 16 have rise elastic members 17 disposed at inward edges in the width direction of the incontinence pad 11. When the incontinence pad 11 is used, the inward edges of the side sheets 16 rise toward the skin of the wearer through contractive force of the rise elastic members 17 to prevent side-leakage of excrement such as urine.

Hereinafter, the present invention will be described in detail by means of examples. However, the present invention is not limited to the examples below, and changes and embodiments that do not depart from the gist of the present invention are included in the scope of the present invention.

«Evaluation methods»

Water absorbent resin powder

(Method for measuring absorption ratio)

Measurement of an absorption ratio is conducted according to JIS K 7223 (1996). A nylon mesh having openings of 63 micrometers (JIS Z8801-1:2000) is cut into a rectangle having a width of 10 cm and a length of 40 cm and folded in half at a center in its longitudinal direction, and both ends thereof are heat-sealed, to produce a nylon bag having a width of 10 cm (inside dimension: 9 cm) and a length of 20 cm. 1.00 g of a measurement sample is precisely weighted and placed into the produced nylon bag such that the sample is uniform at the bottom of the nylon bag. The nylon bag containing the sample is immersed in a saline. After 60 minutes from start of the immersion, the nylon bag is taken out from the saline, and is hung vertically for 1 hour to drain the nylon bag. Then, the mass (F1) of the sample is measured. In addition, the same operation is conducted without using any sample, and a mass F0 (g) at that time is measured. Then, an absorption ratio which is an object is calculated according to the following equation from these masses F1 and F0 and the mass of the sample.

$$\text{Absorption ratio (g/g)} = (F1 - F0) / \text{mass of sample}$$

(Method for measuring water retention amount)

Measurement of a water retention amount is conducted according to JIS K 7223 (1996). A nylon mesh having openings of 63 micrometers (JIS Z8801-1:2000) is cut into a rectangle having a width of 10 cm and a length of 40 cm and folded in half at a center in its longitudinal direction, and both ends thereof are heat-sealed, to produce a nylon bag having a width of 10 cm (inside dimension: 9 cm) and a length of 20 cm. 1.00 g of a measurement sample is precisely weighted and placed into the produced nylon bag such that the sample is uniform at the bottom of the nylon bag. The nylon bag containing the sample is immersed in a saline. After 60 minutes from start of the immersion, the nylon bag is taken out from the saline, and is hung vertically for 1 hour to drain the nylon bag. Then, the nylon bag is dehydrated using a centrifugal hydroextractor (model H-130C special type, manufactured by Kokusan Co., Ltd.). The dehydrating conditions are 143 G (800 rpm) and 2 minutes. A mass (R1) after the dehydration is measured. In addition, the same operation is conducted without using any sample, and a mass R0 (g) at that time is measured. Then, a water retention amount which is an object is calculated according to the following equation from these masses R1 and R0 and the mass

of the sample.

$$\text{Water retention amount (g/g)} = (R1 - R0 - \text{mass of sample}) / \text{mass of sample}$$

(Method for measuring water-absorption speed by a vortex method)

50 mL of a saline (0.9 wt % sodium chloride solution) and a magnetic stir tip (a diameter at center portion: 8 mm, a diameter at both end portions: 7 mm, length: 30 mm, the surface is coated with a fluororesin) are placed into a 100-mL glass beaker, and the beaker is placed on a magnetic stirrer (HPS-100 manufactured by AS ONE Corporation). The rotational speed of the magnetic stirrer is adjusted to 600 plus or minus 60 rpm, and the saline is stirred. 2.0 g of a sample is added to the solution at the center of the vortex of the saline being stirred, and the water-absorption speed (seconds) of the water-absorbent resin powder is measured according to JIS K 7224(1996). Specifically, a stopwatch is started at the time when the addition of the water-absorbent resin powder, which is the sample, to the beaker is completed. The stopwatch is stopped at the time when the stirrer tip is covered with the test solution (the time when the vortex disappears and the surface of the solution becomes flat), and the time (seconds) is recorded as a water-absorption speed. The measurement is conducted five times (n = 5), the highest and lowest values are removed, and the average of the remaining three values is regarded as a measured value. It is noted that these measurements are conducted at 23 plus or minus 2 degrees centigrade and a relative humidity of 50 plus or minus 5%, and samples are stored in the same environment for 24 hours or longer prior to the measurements and then are subjected to the measurements.

(Moisture absorption blocking ratio)

10.0 g of a sample is uniformly placed into an aluminum cup having a bottom diameter of 52 mm and a height of 22 mm (a foil container, product number: 107, manufactured by Toyo Aluminium Ecko Products Co., Ltd.), and the cup is kept still in a constant temperature and humidity chamber at 40 degrees centigrade and a relative humidity of 80% RH for 3 hours. Then, the sample is lightly sieved with a 12-mesh (opening 1.4mm) wire mesh, the mass of powdered matter of the measurement sample that has caused blocking due to moisture absorption and has not passed through the 12 mesh and the mass of the sample that has passed through the 12 mesh are measured, and a moisture absorption blocking ratio which is an object is calculated according to the following equation.

$$\text{Moisture absorption blocking ratio (\%)} = (\text{mass of sample not passing through 12 mesh after being kept still}) / (\text{mass of sample not passing through 12 mesh after being kept still} + \text{mass of sample passing through 12 mesh after being kept still}) \times 100$$

The measurement is conducted five times (n = 5), the highest and lowest values are removed, and the average of the remaining three values is regarded as a measured value. It is noted that these measurements are conducted at 23 plus or minus 2 degrees centigrade and a relative humidity of 50 plus or minus 5%, and samples are stored in the same environment for 24 hours or longer prior to the measurements and then are subjected to the measurements.

(Method for measuring liquid-passing rate under load)

In a 100-mL glass beaker, 0.32 plus or minus 0.005 g of a water-absorbent resin powder that is a sample is immersed in 100 mL of a saline (0.9 wt% sodium chloride solution) and allowed to stand for 60 minutes, thereby swelling the water-absorbent resin powder. Separately, a filtration cylindrical tube is prepared in which a wire mesh (openings: 150 micrometers, a bio-column sintered stainless steel filter 30SUS sold by Sansyo Co., Ltd) and a narrow tube (inner diameter: 4 mm, length: 8 cm) equipped with a cock (inner diameter: 2 mm) are provided at the lower end of an opening portion of a cylinder (inner diameter: 25.4 mm) that stands vertically. All the content within the beaker including the swollen measurement sample is placed into the cylindrical tube in a state where the cock is closed. Next, a cylindrical bar that has a diameter of 2 mm and has, at its end, a wire mesh having openings of 150 micrometers and a diameter of 25 mm is inserted into the filtration cylindrical tube such that the wire mesh comes into contact with the measurement sample, and further a weight is placed such that a load of 2.0 KPa is applied to the measurement sample. In this state, the filtration cylindrical tube is allowed to stand for 1 minute. Then, the cock is opened to allow the solution to flow out, and the time ($T_1$) (seconds) taken until the solution level within the filtration cylindrical tube reaches a 40-mL scale mark from a 60-mL scale mark (i.e., 20 mL of the solution passes) is measured. A liquid-passing rate under a load of 2.0 KPa is calculated from the following equation using the measured time $T_1$ (seconds). It is noted that in the equation, $T_0$ (seconds) is a measured value of a time taken for 20 mL of a saline to pass through the wire mesh in a state where no measurement sample was put in the filtration cylindrical tube.

$$\text{Liquid-passing rate under load (seconds)} = (T_1 - T_0)$$

Diffusibility improvement material

(Method for measuring under-load liquid passing rate)

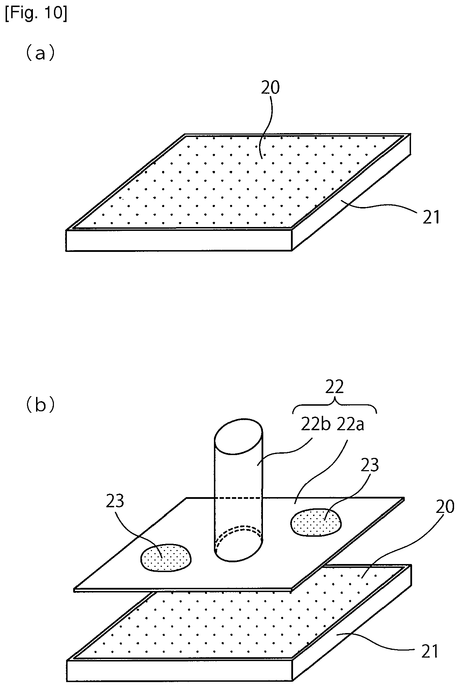

FIG. 10 is a schematic diagram for describing a method for measuring an under-load liquid passing rate of the diffusibility improvement material. As shown in (a) of FIG. 10, a diffusibility improvement material 20 which was used as a sample was arranged such that its planar view shape became a square shape whose side was larger than 100 mm. It should be noted that, when a particulate diffusibility improvement material was used, a frame 21 for immobilizing the diffusibility improvement material was provided, and the diffusibility improvement material was arranged within the frame 21. At this moment, the particulate diffusibility improvement material was arranged to become a single layer in the thickness direction and make gaps between particles in a planar direction become as small as possible. When a sheet-like diffusibility improvement material was used, a sheet that was actually used as the absorbent body was cut out such that its planar view shape was a square shape whose side was larger than 100 mm. Then, the diffusibility improvement material within the frame was immersed and kept in saline solution (0.9 mass% sodium chloride solution) for 60 minutes.

As shown in (b) of FIG. 10, a measuring jig 22 includes a stainless steel plate 22a (100 mm x 100 mm) having an opening (internal diameter 25.4 mm) at its center, and a cylinder 22b (internal diameter 25.4 mm) attached perpendicularly to the opening portion. The measuring jig 22 was placed on the diffusibility improvement agent 20. A weight 23 was placed on the measuring jig 22 such that a load of 2.0 kPa was applied on a measured sample, and the sample was kept in this state for 1 minute. Then, 150 ml of saline solution was poured in the cylinder, and the time (T) (seconds) from the start of pouring the saline solution until the liquid in the cylinder disappeared was measured, and this time was used as the under-load liquid passing rate. The measurement was conducted by n=5, and value points at the top and bottom were each deleted, and an average of the remaining three points was used as a measured value. It should be noted that the measurements were conducted at 23 plus or minus 2 degrees centigrade and at a relative humidity of 50 plus or minus 5%, and a measurement was conducted after a sample was stored at the same environment for 24 hours or longer.

Manufacturing of absorbent body

Absorbent body 1-1

After applying a synthetic-rubber based hot melt adhesive on a spunbond nonwoven fabric, a mixture of pulp and a water absorbent resin powder ("Aqua Pearl DS560," manufactured by San-Dia Polymers, Ltd.) was sprayed thereon to form a water absorption layer only having a water absorption region. The planar view shape of the water absorption layer was a rectangular shape in which the length in the longitudinal direction was 40 cm and the length in the width direction was 10 cm. The central part in the width direction of the water absorption layer was cut out in a substantially rectangular shape in planar view. ABS resin particles (particle diameter: 6 mm, under-load liquid passing rate: 10 seconds) were sprayed as a diffusibility improvement material on the cut-out portion to form a diffusion-and-water absorption layer including a diffusion region and a water absorption region. It should be noted that the diffusion region was formed from a central part in the longitudinal direction of the diffusion-and-water absorption layer toward one end side in the longitudinal direction, so as to span over the central line of the absorbent body in the width direction and such that the central line of the absorbent body in the width direction and the central line of the diffusion region in the width direction match each other. The diffusion region had a width of 1.0 cm, a length of 40 cm, and an area ratio with respect to the whole diffusion-and-water absorption layer of 10%. An air-through nonwoven fabric was laminated on the diffusion-and-water absorption layer, and the obtained laminated body was pressed to obtain an absorbent body 1-1 consisting of a single diffusion-and-water absorption layer. With regard to the physical properties of the water absorbent resin powder, the water absorption ratio was 60 g/g, the water retention amount was 42 g/g, the water absorption speed determined by a vortex method was 38 seconds, the moisture absorption blocking rate was 0.1%, and the under-load liquid passing rate was 5 seconds.

Absorbent bodies 1-2, 1-3, 1-4

Absorbent bodies 1-2, 1-3, and 1-4 were obtained in a manner similar to the manufacturing of the absorbent body 1-1, except for changing the width of the diffusion region. The diffusion region of the absorbent body 1-2 had a width of 2.7 cm, a length of 40 cm, and an area ratio with respect to the whole diffusion-and-water absorption layer of 27%. The diffusion region of the absorbent body 1-3 had a width of 5.0 cm, a length of 40 cm, and an area ratio with respect to the whole diffusion-and-water absorption layer of 50%. The diffusion region of the absorbent body 1-4 had a width of 7.0 cm, a length of 40 cm, and an area ratio with respect to the whole diffusion-and-water absorption layer of 70%.

Absorbent body 2-1

After applying a synthetic-rubber based hot melt adhesive on a spunbond nonwoven fabric, a mixture of pulp and a water absorbent resin powder ("Aqua Pearl DS560," manufactured by San-Dia Polymers, Ltd.) was sprayed thereon to form a water absorption layer only having a water absorption region. The planar view shape of the water absorption layer was a rectangular shape, in which the length in the longitudinal direction was 40 cm and the length in the width direction was 10 cm. After laminating a spunbond nonwoven fabric on the water absorption layer and applying a synthetic-rubber based hot melt adhesive thereon, ABS resin particles (particle diameter: 6 mm, under-load liquid passing rate: 10 seconds) were uniformly sprayed on the whole top surface of the water absorption layer as a diffusibility improvement material to form a diffusion layer only having a diffusion region. The planar view shape of the diffusion layer was a rectangular shape, in which the length in the longitudinal direction was 40 cm, the length in the width direction was 10 cm, and the thickness was 8 mm. After laminating a spunbond nonwoven fabric on the diffusion layer and applying a synthetic-rubber based hot melt adhesive thereon, an air-through nonwoven fabric was further laminated thereon and the obtained laminated body was pressed to obtain the absorbent body 2-1 including the diffusion layer as the uppermost layer and the water absorption layer as a lower layer adjacent to the uppermost layer.

Comparative absorbent body 1

After applying a synthetic-rubber based hot melt adhesive on a spunbond nonwoven fabric, a mixture of pulp and a water absorbent resin powder was sprayed thereon to form a water absorption layer only having a water absorption region. An air-through nonwoven fabric was laminated on the water absorption layer, and the obtained laminated body was pressed to obtain a comparative absorbent body 1 consisting of a single water absorption layer.

Comparative absorbent body 2

After applying a synthetic-rubber based hot melt adhesive on a spunbond nonwoven fabric, a mixture of pulp and a water absorbent resin powder was sprayed thereon, and one part thereof was cut out in a substantially rectangular shape in planar view (area ratio with respect to the whole water absorption layer: 10%). A water absorption layer was formed by spraying the water absorbent resin powder to the part that was cut out. An air-through nonwoven fabric was laminated on the water absorption layer, and the obtained laminated body was pressed to obtain a comparative absorbent body 2 consisting of a single water absorption layer.

Comparative absorbent body 3

After applying a synthetic-rubber based hot melt adhesive on a spunbond nonwoven fabric, a mixture of pulp and a water absorbent resin powder was sprayed thereon to form a first water absorption layer only having a water absorption region. After laminating a spunbond nonwoven fabric on the first water absorption layer and applying a synthetic-rubber based hot melt adhesive thereon, the water absorbent resin powder was sprayed thereon to form a second water absorption layer. After laminating a spunbond nonwoven fabric on the second water absorption layer and applying a synthetic-rubber based hot melt adhesive thereon, an air-through nonwoven fabric was laminated thereon and the obtained laminated body was pressed to obtain a comparative absorbent body 3 having two water absorption layers.

The rate of absorbing artificial urine and dryness value were measured for the obtained absorbent bodies 1 and 2 and comparative absorbent bodies 1 to 3, and the results are shown in Table 1. The measurements were conducted as described below.

<Artificial urine absorption rate, surface dryness value determined by SDME method>

An absorbent body was immersed and kept in artificial urine (0.03 mass% potassium chloride, 0.08 mass% magnesium sulfate, 0.8 mass% sodium chloride, and 99.09 mass% deionized water) for 60 minutes for preparation, and a sufficiently wet absorbent body was produced. In addition, an absorbent body was heated and dried at 80 degrees centigrade for 2 hours to produce a sufficiently dried absorbent body. A detector of an SDME (Surface Dryness Measurement Equipment) tester (manufactured by WK system Co., Ltd.) was placed on (at the center in the width direction and center in the length direction) the sufficiently wet absorbent body to configure a 100% dryness value. Next, the detector of the SDME tester was placed on (at the center in the width direction and center in the length direction) the sufficiently dried absorbent body to configure a 0% dryness, and calibration of the SDME tester was conducted. Next, a metal ring (internal diameter 70 mm, length 50 mm) was set at the center of the absorbent body that is to be measured, and 20 ml of artificial urine was poured thereto and the time required until the artificial urine was absorbed was measured to obtain an absorption rate. The metal ring was removed immediately after the absorption had ended, and the SDME detector was placed at the center of the absorbent body to start measuring the surface dryness value. A value obtained 1 minute after the start of the measurement was used as a surface dryness value. After the absorbent body was kept for 30 minutes, artificial urine was poured thereto for the second time. The operation was conducted similarly to the operation conducted the first time, and the absorption rate of the second time and the surface dryness value of the second time were obtained. The measurements were conducted three times (n=3), and an average of the three measurements was used as a measured value. It should be noted that the artificial urine, the measurement atmosphere, and the keeping atmosphere were 25 plus or minus 5 degrees centigrade and 65 plus or minus 10% RH.

[Table 1]

| | Area ratio of diffusion region (%) | Absorption rate | | | Dryness value | | |
|---|---|---|---|---|---|---|---|
| | | First measurement | Second measurement | Third measurement | First measurement | Second measurement | Third measurement |
| Absorbent body 1-1 | 10 | 22 seconds | 30 seconds | 42 seconds | 20% | 33% | 40% |
| Absorbent body 1-2 | 27 | 22 seconds | 22 seconds | 30 seconds | 23% | 38% | 54% |
| Absorbent body 1-3 | 50 | 22 seconds | 22 seconds | 30 seconds | 20% | 42% | 66% |
| Absorbent body 1-4 | 70 | 22 seconds | 30 seconds | 42 seconds | 20% | 50% | 66% |
| Absorbent body 2-1 | - | 20 seconds | 25 seconds | 25 seconds | 18% | 20% | 25% |
| Comparative absorbent body 1 | - | 35 seconds | 120 seconds | 300 seconds | 22% | 44% | 90% |
| Comparative absorbent body 2 | - | 30 seconds | 180 seconds | 300 seconds | 20% | 72% | 90% |
| Comparative absorbent body 3 | - | 34 seconds | 195 seconds | 300 seconds | 22% | 64% | 90% |

As can be understood from Table 1, the absorbent bodies 1-1 to 1-4, and 2-1, each having a layer on which a diffusion region is formed at least at one part thereof, have a fast absorption rate and excellent dryness. This is thought to be caused by an improvement in diffusibility of the artificial urine in the absorbent body due to the sprayed diffusibility improvement material.

On the other hand, the comparative absorbent bodies 1 to 3 are cases where there are no layers having a diffusion region formed thereon, and those have a slow absorption rate and are inferior in terms of dryness. Since the comparative absorbent bodies 1 to 3 do not have a layer with a diffusion region formed thereon, gel-blocking occurs easily when the water absorbent resin powder absorbs water, and permeability to the absorbent body and dryness are unlikely to improve. This is thought to be the reason why the absorption rate in the first measurement, the absorption rate in the second measurement, and the dryness in the second measurement and beyond were inferior.

The present invention includes the following embodiments.

<Embodiment 1>

An absorbent body including a water absorbent resin powder, the absorbent body comprising:

a layer having formed at least partially thereon a diffusion region where a diffusibility improvement material having an under-load liquid passing rate of 15 seconds or less is disposed.

<Embodiment 2>

The absorbent body according to embodiment 1, wherein the layer includes a diffusion-and-water absorption layer including the diffusion region and a water absorption region where the water absorbent resin powder is disposed.

<Embodiment 3>

The absorbent body according to embodiment 1 or 2, wherein an area ratio of the diffusion region with respect to the surface of the diffusion-and-water absorption layer ranges from 5% to 70%.

<Embodiment 4>

The absorbent body according to any one of embodiments 1 to 3, wherein the diffusion region is formed in a circular shape, an elliptical shape, a polygonal shape, a polygonal shape having rounded corners, or a slit shape.

<Embodiment 5>

The absorbent body according to embodiment 4, wherein the diffusion region having the circular shape, the elliptical shape, the polygonal shape, or the polygonal shape having rounded corners is formed starting from an approximately center part of the diffusion-and-water absorption layer in a longitudinal direction, toward one side thereof in the longitudinal direction.

<Embodiment 6>

The absorbent body according to any one of embodiments 1 to 5, essentially consisting of a single layer of the diffusion-and-water absorption layer.

<Embodiment 7>

The absorbent body according to embodiment 1, comprising at least two layers of a diffusion layer only having a diffusion region and a water absorption layer only having a water absorption region below the diffusion layer.

<Embodiment 8>

The absorbent body according to embodiment 7, wherein the water absorption layer includes a water absorbent resin powder satisfying the following requirements:

Absorption ratio: 50 g/g to 70 g/g
Water retention amount: 25 g/g to 60 g/g.

<Embodiment 9>

The absorbent body according to embodiment 7, wherein the water absorption layer includes a water absorbent resin powder whose absorption rate determined by a vortex method is from 5 seconds to 60 seconds.

<Embodiment 10>

The absorbent body according to embodiment 1, comprising at least two layers of a diffusion-and-water absorption layer having formed thereon a slit shaped diffusion region and a water absorption layer only having a water absorption region below the diffusion-and-water absorption layer.

<Embodiment 11>

The absorbent body according to embodiment 10, wherein the water absorption layer includes a water absorbent resin powder satisfying the following requirements:

Absorption ratio: 40 g/g to 70 g/g
Water retention amount: 30 g/g to 50 g/g.

<Embodiment 12>

An absorbent article comprising the absorbent body according to any one of embodiments 1 to 11.

**Industrial Applicability**

The absorbent body of the present invention can be suitably used as, for example, an absorbent article used for absorbing body fluid excreted from the human body, and can be used particularly suitably as absorbent articles such as incontinence pads, disposable diapers, sanitary napkins, and breast-milk pads.

**Reference Signs List**

1: absorbent body, 2: diffusion region, 3: water absorption region, 4: diffusion-and-water absorption layer, 5: diffusion layer, 6: water absorption layer, 11: incontinence pad (absorbent article), 12: top sheet, 13: back sheet, 16: side sheet, 17: rise elastic member, 20: diffusibility improvement material, 21: frame, 22: measuring jig, 22a: stainless steel plate, 22b: cylinder, 23: weight

**Claims**

1. An absorbent body (1) including a water absorbent resin powder, the absorbent body (1) comprising:

    a layer (4, 5) having formed at least partially thereon a diffusion region (2) where a diffusibility improvement material having an under-load liquid passing rate, determined by the test method described herein, of 15 seconds or less is disposed, wherein the diffusibility improvement material is resin particles having a particle diameter of from 0.05 mm to 10 mm.

2. The absorbent body (1) according to claim 1, wherein the layer (4, 5) includes a diffusion-and-water absorption layer (4) including the diffusion region (2) and a water absorption region (3) where the water absorbent resin powder is disposed.

3. The absorbent body (1) according to claim 2, wherein an area ratio of the diffusion region (2) with respect to the surface of the diffusion-and-water absorption layer (4) ranges from 5% to 70%.

4. The absorbent body (1) according to claim 1, wherein the diffusion region (2) is formed in a circular shape, an elliptical shape, a polygonal shape, a polygonal shape having rounded corners, or a slit shape.

5. The absorbent body (1) according to claim 2, wherein the diffusion region (2) having a circular shape, an elliptical shape, a polygonal shape, or a polygonal shape having rounded corners is formed starting from an approximately center part of the diffusion-and-water absorption layer (4) in a longitudinal direction, toward one side thereof in the longitudinal direction.

6. The absorbent body (1) according to claim 2, essentially consisting of a single layer of the diffusion-and-water absorption layer (4).

7. The absorbent body (1) according to claim 1, comprising at least two layers of a diffusion layer (5) only having a diffusion region (2) and a water absorption layer (6) only having a water absorption region (3) below the diffusion layer (5).

8. The absorbent body (1) according to claim 7, wherein the water absorption layer (5) includes a water absorbent resin powder satisfying the following requirements:

   Absorption ratio: 50 g/g to 70 g/g
   Water retention amount: 25 g/g to 60 g/g.

9. The absorbent body (1) according to claim 7, wherein the water absorption layer (5) includes a water absorbent resin powder whose absorption rate determined by a vortex method is from 5 seconds to 60 seconds.

10. The absorbent body (1) according to claim 2, comprising at least two layers of a diffusion-and-water absorption layer (4) having formed thereon a slit shaped diffusion region (2) and a water absorption layer (6) only having a water absorption region (3) below the diffusion-and-water absorption layer (4).

11. The absorbent body (1) according to claim 10, wherein the water absorption layer (6) includes a water absorbent resin powder satisfying the following requirements:

   Absorption ratio: 40 g/g to 70 g/g
   Water retention amount: 30 g/g to 50 g/g.

12. An absorbent article (11) comprising the absorbent body (1) according to any one of claims 1 to 11.


**Revendications**

1. Corps absorbant (1) incluant une poudre de résine absorbant l'eau, le corps absorbant (1) comprenant :

   une couche (4, 5) sur laquelle, tout au moins en partie, est formée une région de diffusion (2) dans laquelle est disposé un matériau d'amélioration de la diffusibilité ayant une vitesse de passage du liquide sous charge, déterminée par la méthode d'essai décrite ici, de 15 secondes ou moins, où le matériau d'amélioration de la diffusibilité correspond à des particules de résine d'un diamètre particulaire qui va de 0,05 mm à 10 mm.

2. Corps absorbant (1) selon la revendication 1, où la couche (4, 5) inclut une couche de diffusion et d'absorption de l'eau (4) incluant la région de diffusion (2) et une région d'absorption de l'eau (3) dans laquelle est disposée la poudre de résine absorbant l'eau.

3. Corps absorbant (1) selon la revendication 2, où un rapport entre la surface de la région de diffusion (2) et la surface de la couche de diffusion et d'absorption de l'eau (4) va de 5% à 70%.

4. Corps absorbant (1) selon la revendication 1, où la région de diffusion (2) est formée en une forme circulaire, une forme elliptique, une forme polygonale, une forme polygonale à coins arrondis ou une forme de fente.

5. Corps absorbant (1) selon la revendication 2, où la région de diffusion (2) ayant une forme circulaire, une forme elliptique, une forme polygonale, une forme polygonale à coins arrondis est formée en débutant à partir d'une partie approximativement centrale de la couche de diffusion et d'absorption de l'eau (4) dans une direction longitudinale, vers un côté de celle-ci dans la direction longitudinale.

6. Corps absorbant (1) selon la revendication 2 qui consiste essentiellement en une couche unique de la couche de diffusion et d'absorption de l'eau (4).

7. Corps absorbant (1) selon la revendication 1 qui comprend au moins deux couches correspondant à une couche de diffusion (5) ayant uniquement une région de diffusion (2) et une couche d'absorption de l'eau (6) ayant uniquement une région d'absorption de l'eau (3) en dessous de la couche de diffusion (5).

8. Corps absorbant (1) selon la revendication 7, où la couche d'absorption de l'eau (5) inclut une poudre de résine absorbant l'eau qui satisfait les exigences suivantes :

   Rapport d'absorption : de 50 g/g à 70 g/g
   Capacité de rétention de l'eau : de 25 g/g à 60 g/g.

9. Corps absorbant (1) selon la revendication 7, où la couche d'absorption de l'eau (5) inclut une poudre de résine absorbant l'eau dont la vitesse d'absorption, déterminé par une méthode vortex, va de 5 secondes à 60 secondes.

10. Corps absorbant (1) selon la revendication 2 qui comprend au moins deux couches correspondant à une couche de diffusion et d'absorption de l'eau (4) sur laquelle est formée une région de diffusion en forme de fente (2) et une couche d'absorption de l'eau (6) ayant uniquement une région d'absorption de l'eau (3) en dessous de la couche de diffusion et d'absorption de l'eau (4).

11. Corps absorbant (1) selon la revendication 10, où la couche d'absorption de l'eau (6) inclut une poudre de résine absorbant l'eau qui satisfait les exigences suivantes :

    Rapport d'absorption : de 40 g/g à 70 g/g
    Capacité de rétention de l'eau : de 30 g/g à 50 g/g.

12. Article absorbant (11) comprenant le corps absorbant (1) selon l'une quelconque des revendications 1 à 11.

**Patentansprüche**

1. Absorbierender Körper (1) mit einem wasserabsorbierenden Harzpulver, wobei der absorbierende Körper (1) Folgendes umfasst:

   eine Schicht (4, 5), auf der zumindest teilweise ein Diffusionsbereich (2) ausgebildet ist, in dem ein Diffusionsfähigkeitsverbesserungsmaterial mit einer durch das hier beschriebene Testverfahren bestimmten Belastungs-Flüssigkeitsdurchlassrate von 15 Sekunden oder weniger angeordnet ist, wobei das Diffusionsfähigkeitsverbesserungsmaterial Harzteilchen mit einem Teilchendurchmesser von 0,05 mm bis 10 mm sind.

2. Absorbierender Körper (1) nach Anspruch 1, wobei die Schicht (4, 5) eine Diffusions- und Wasserabsorptionsschicht (4) umfasst, die den Diffusionsbereich (2) und einen Wasserabsorptionsbereich (3), in dem das wasserabsorbierende Harzpulver angeordnet ist, umfasst.

3. Absorbierender Körper (1) nach Anspruch 2, wobei ein Flächenverhältnis des Diffusionsbereichs (2) bezogen auf die Oberfläche der Diffusions- und Wasserabsorptionsschicht (4) von 5 % bis 70 % reicht.

4. Absorbierender Körper (1) nach Anspruch 1, wobei der Diffusionsbereich (2) in einer Kreisform, einer elliptischen Form, einer polygonalen Form, einer polygonalen Form mit abgerundeten Ecken oder einer Schlitzform ausgebildet ist.

5. Absorbierender Körper (1) nach Anspruch 2, wobei der Diffusionsbereich (2) mit einer Kreisform, einer elliptischen Form, einer polygonalen Form oder einer polygonalen Form mit abgerundeten Ecken ausgehend von einem in Längsrichtung etwa mittigen Teil der Diffusions- und Wasserabsorptionsschicht (4) hin zu einer Seite derselben in der Längsrichtung ausgebildet ist.

6. Absorbierender Körper (1) nach Anspruch 2, der im Wesentlichen aus einer einzigen Schicht der Diffusions- und Wasserabsorptionsschicht (4) besteht.

7. Absorbierender Körper (1) nach Anspruch 1, umfassend zumindest zwei Schichten einer Diffusionsschicht (5), die nur einen Diffusionsbereich (2) aufweist, und eine Wasserabsorptionsschicht (6), die nur einen Wasserabsorptionsbereich (3) aufweist, unterhalb der Diffusionsschicht (5).

8. Absorbierender Körper (1) nach Anspruch 7, wobei die Wasserabsorptionsschicht (5) ein wasserabsorbierendes Harzpulver umfasst, das die folgenden Anforderungen erfüllt: Absorptionsverhältnis: 50 g/g bis 70 g/g Wasserrückhaltemenge: 25 g/g bis 60 g/g.

9. Absorbierender Körper (1) nach Anspruch 7, wobei die Wasserabsorptionsschicht (5) ein wasserabsorbierendes Harzpulver umfasst, dessen durch ein Vortexverfahren bestimmte Absorptionsrate 5 Sekunden bis 60 Sekunden beträgt.

10. Absorbierender Körper (1) nach Anspruch 2, umfassend zumindest zwei Schichten einer Diffusions- und Wasserabsorptionsschicht (4), auf der ein schlitzförmiger Diffusionsbereich (2) ausgebildet ist, und eine Wasserabsorptionsschicht (6), die nur einen Wasserabsorptionsbereich (3) aufweist, unterhalb der Diffusions- und Wasserabsorptionsschicht (4).

11. Absorbierender Körper (1) nach Anspruch 10, wobei die Wasserabsorptionsschicht (6) ein wasserabsorbierendes Harzpulver umfasst, das die folgenden Anforderungen erfüllt: Absorptionsverhältnis: 40 g/g bis 70 g/g Wasserrückhaltemenge: 30 g/g bis 50 g/g.

12. Absorbierender Artikel (11), der den absorbierenden Körper (1) nach einem der Ansprüche 1 bis 11 umfasst.

[Fig. 1]

[Fig. 2]

[Fig. 3]

[Fig. 4]

[Fig. 5]

[Fig. 6]

[Fig. 7]

[Fig. 8]

16  17  12  13                    11

                                    1

V                                V

A        B

[Fig. 9]

17              12              17

16                                  16

                    13    2  3

                    4

[Fig. 10]

（a）

（b）

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011055959 A
- JP 2002528302 A
- JP 2006524112 A
- EP 1518567 A1
- EP 1579831 A1
- US 20040019342 A1
- US 5419956 A

- JP 3648553 B
- JP 2003165883 A
- JP 2005075982 A
- JP 2005095759 A
- JP 2003225565 A
- JP 2006131767 A

**Non-patent literature cited in the description**

- Perry's Chemical Engineers Handbook. The Mc-Graw-Hill Companies, 1984, 21